# EUROPEAN PATENT APPLICATION

(11) **EP 4 394 099 A1**
(43) Date of publication of application: **03.07.2024**
(21) Application number: 22867474.3
(22) Date of filing: 13.09.2022
(51) Int. Cl.: D01F 4/02, D06M 15/15, A61L 9/01

(54) **DEODORANT MATERIAL, AGENT FOR IMPARTING DEODORANT PROPERTIES, AND METHOD FOR IMPARTING DEODORANT PROPERTIES**

(30) Priority: 13.09.2021 JP 2021148844
(71) Applicant: Spiber Inc., Tsuruoka-shi, Yamagata 997-0052 (JP); Goldwin Inc., Toyama 932-0112 (JP)
(72) Inventor: SATO Akito, Tsuruoka-shi, Yamagata 997-0052 (JP); KOJIMA Takayuki, Oyabe-shi, Toyama 932-0112 (JP)
(74) Representative: Handley, Matthew Edward
(86) International application number: PCT/JP2022/034268
(87) International publication number: WO 2023/038153

(57) **Abstract**

The present invention provides a deodorant material containing artificial fibroin as an active component, an agent for imparting deodorant properties, containing artificial fibroin as an active component, an article containing the agent for imparting deodorant properties, and a method for imparting deodorant properties, including a step of adding artificial fibroin to an article.

## Description

### Technical Field

The present invention relates to a deodorant material, an agent for imparting deodorant properties, and a method for imparting deodorant properties.

### Background Art

A deodorant is utilized in a variety of fields, and can maintain a high commercial value of an article by reducing malodor emitted from the article. Therefore, many deodorants have been developed so far. As the deodorant, a conjugated fiber spinning stock solution containing a crosslinked protein obtained by adding a water-soluble crosslinking agent to an alkali-soluble protein and cellulose (Patent Literature 1), a fecal odor improver containing a whey protein (Patent Literature 2), a hair foam composition containing a hydrolyzed protein bonded to platinum (Patent Literature 3), a deodorant containing γ-PGA and/or a crosslinked product thereof (Patent Literature 4), regenerated silk obtained by subjecting silk-derived fibroin to a specific treatment (Patent Literature 5), a deodorant containing at least one zinc compound selected from zinc oxide and zinc carbonate and at least one amino acid selected from glycine, alanine, phenylalanine, a glutamate, and sarcosine (Patent Literature 6), and the like are known.

However, some of these deodorants cannot be used for what directly touches a human skin or the like, and some of these deodorants have no biodegradability and thus may cause environmental pollution by disposal after use. The fecal odor improver described in Patent Literature 2 can solve the above problems, but has a problem that it is difficult to maintain stable quality all the time.

### Citation List

### Patent Literatures

Patent Literature 1: JP 2004-149953 A
Patent Literature 2: JP 2006-124342 A
Patent Literature 3: JP 2012-56850 A
Patent Literature 4: JP 2005-81040 A
Patent Literature 5: JP 2013-245427 A

### Summary of Invention

### Technical Problem

Therefore, an object of the present invention is to provide a deodorant material having biodegradability. Another object of the present invention is a method capable of imparting deodorant properties to a predetermined article.

### Solution to Problem

The present invention provides the following [1] to [11] .
[1] A deodorant material containing artificial fibroin as an active component.
[2] The deodorant material according to [1], in which the artificial fibroin contains artificial spider silk fibroin.
[3] The deodorant material according to [1] or [2], which is in a form of a fiber.
[4] A covering containing the deodorant material according to [3].
[5] An agent for imparting deodorant properties, containing artificial fibroin as an active component.
[6] The agent for imparting deodorant properties according to [5], in which the artificial fibroin contains artificial spider silk fibroin.
[7] The agent for imparting deodorant properties according to [5] or [6], which is in a form of a fiber.
[8] An article containing the agent for imparting deodorant properties according to any one of [5] to [7].
[9] The article according to [8], in which the content of the agent for imparting deodorant properties is more than 3% by mass with respect to the total weight of the article.
[10] A method for imparting deodorant properties, including a step of adding artificial fibroin to an article.
[11] The method for imparting deodorant properties according to [10], in which the artificial fibroin is added in a form of a composition containing the artificial fibroin.

### Advantageous Effects of Invention

As described later, a deodorant material according to the present invention can be artificially produced, can also be applied to mass production, and has biodegradability, and therefore has excellent safety for use and a small environmental load. By using the deodorant material according to the present invention as a material of an existing product, it is possible to reduce use of artificial fibers having a large environmental load and to suppress a problem of environmental destruction due to collection of natural fibers whose mass production is difficult. An agent for imparting deodorant properties according to the present invention can impart deodorant properties to a target product by a simple process.

### Brief Description of Drawings

Fig. 1 is a schematic diagram illustrating an example of a domain sequence of artificial fibroin.
Fig. 2 is a diagram illustrating a distribution of values of z/w (%) in naturally derived fibroins.
Fig. 3 is a diagram illustrating a distribution of values of x/y (%) in naturally derived fibroins.
Fig. 4 is a schematic diagram illustrating an example of a domain sequence of artificial fibroin.
Fig. 5 is a schematic diagram illustrating an example of a domain sequence of artificial fibroin.
Fig. 6 is an explanatory diagram schematically illustrating an example of a spinning apparatus for producing an artificial fibroin filament.
Fig. 7 is a graph illustrating results of evaluating deodorant properties of a fiber A.
Fig. 8 is a graph illustrating deodorant effects of fibers B to G when the fibers B to G are unwashed.
Fig. 9 is a graph illustrating deodorant effects of the fibers B to G after 10 times of washing.
Fig. 10 is a graph illustrating deodorant effects of the fibers B to G after 100 times of washing.
Fig. 11 is a graph illustrating deodorant effects of the fibers B to G one month after 100 times of washing.

### Description of Embodiments

Hereinafter, embodiments of the present invention will be described in detail.

### [First Embodiment]

A first embodiment of the present invention is a deodorant material containing artificial fibroin as an active component. The amount of artificial fibroin in the deodorant material may be any amount that can exhibit a deodorant effect of artificial fibroin, and is, for example, 10% by mass or more on the basis of the total mass of the deodorant material. The amount of artificial fibroin is preferably 20% by mass or more, 30% by mass or more, 40% by mass or more, 50% by mass or more, 60% by mass or more, 70% by mass or more, 75% by mass or more, 80% by mass or more, 85% by mass or more, 90% by mass or more, 95% by mass or more, or 97% by mass or more on the basis of the total mass of the deodorant material from a viewpoint of further exhibiting a deodorant effect. The deodorant material may consist of artificial fibroin.

The deodorant material according to the present embodiment is not limited as long as it is a material exhibiting a deodorant effect. The shape of the deodorant material is not limited as long as artificial fibroin can exhibit a deodorant effect. The form of the deodorant material may be, for example, a liquid (for example, a solution or a suspension), a fiber, a powder, a plate, a film, a sheet, a gel, a porous body, a particle, or the like, and the shape thereof may be, for example, a cube, a rectangular parallelepiped, a triangular pyramid, a triangular prism, a cylinder, or a sphere. In addition, these forms may be combined to form a braid, a fabric (for example, a woven fabric, a nonwoven fabric, or a knitted fabric), a covering (for example, a shirt, an undergarment, a muffler, a sock, an apron, a tie, a jacket, a coat, a glove, or a hat), bedding (for example, a sheet, a comforter, a sheet cover, a comforter cover, or a towel), or a back portion or a seat surface of a chair or a vehicle seat. For example, a fibrous deodorant material may be mixed with a fiber constituting a braid or a fabric and used. The knitted fabric may be a fabric knitted by plain knitting, interlock knitting, rib knitting, or the like. Note that when the deodorant material according to the present embodiment is in a form other than a liquid, the size (thickness, area, particle size, mass, and the like) of the deodorant material is appropriately adjusted or selected according to the type of the form, the size of a target deodorant effect, and the like.

In an embodiment, the deodorant material is a single yarn consisting only of an artificial fibroin fiber, a composite yarn obtained by combining an artificial fibroin fiber with other fibers, or a yarn in which the single yarn and the composite yarn are combined. The artificial fibroin fiber and other fibers constituting the single yarn or the composite yarn may be filaments (long fibers) or staples (short fibers). The composite yarn contains artificial fibroin as a main component. As the other fibers, a natural fiber, a regenerated fiber, a synthetic fiber, and the like are used. The form of the single yarn or the composite yarn is not particularly limited, and may be a spun yarn, a twisted yarn, a false twisted yarn, a bundle of untwisted filaments, a cover yarn, or the like. The artificial fibroin fiber may contain components and contaminants other than artificial fibroin, such as a protein other than artificial fibroin and a predetermined additive.

A shrinkage ratio of the artificial fibroin fiber when the artificial fibroin fiber is brought into contact with an aqueous medium may be more than 7%, 15% or more, 25% or more, 32% or more, 40% or more, 48% or more, 56% or more, 64% or more, or 72% or more. The shrinkage ratio may be, for example, 80% or less, 70% or less, 60% or less, or 50% or less. Here, the shrinkage ratio of the artificial fibroin fiber is defined by the following formula. Shrinkage ratio = {1 - (lenght of artificial fibroin fiber after contact with aqueous medium / lenght of artificial fibroin fiber before contact with aqueous medium)} × 100(%)

The artificial fibroin fiber may be an artificial fibroin crimped fiber having a crimp. The degree of crimping is not limited, and the number of crimps may be, for example, 10 to 100 crimps/40 mm or 20 to 40 crimps/40 mm.

The artificial fibroin fiber preferably has excellent flame retardancy, and a critical oxygen index (LOI) value thereof may be 18 or more, 20 or more, 22 or more, 24 or more, 26 or more, 28 or more, 29 or more, or 30 or more. In the present specification, the LOI value is a value measured in accordance with the "test method of a particulate or low-melting point synthetic resin" described in "Notice No. 50 of the Office of Hazardous Materials Regulation (May, 31, 1995).

The artificial fibroin fiber may contain hydrophilic artificial fibroin and/or may contain artificial fibroin having an average hydropathy index (average HI) of 0 or less in order to exhibit sufficient flame retardancy.

The artificial fibroin fiber preferably has excellent hygroscopic exothermicity, and a maximum hygroscopic exothermic degree thereof determined according to the following formula A may be more than 0.025°C/g. Here, the sample in formula A refers to the artificial fibroin fiber. the sample temperature reaches an equilibrium, and then maximum hygroscopic exothermic degree = {(maximum value of a sample temperature when a sample is placed in a low humidity environment until the sample temperature reaches an equilibrium, and then transferred to a high humidity environment) - (a sample temperature when the sample is placed in a low humidity environment until the sample temperature reaches an equilibrium, and then transferred to a high humidity environment)} (°C) / sample weight (g) [in which the low humidity environment means an environment with a temperature of 20°C and a relative humidity of 40%, and the hight humidity environment means an environment with a temperature of 20°C and a relative humidity of 90%.]

The maximum hygroscopic exothermic degree of the artificial fibroin fiber may be 0.026°C/g or more, 0.027°C/g or more, 0.028°C/g or more, 0.029°C/g or more, 0.030°C/g or more, 0.035°C/g or more, or 0.040°C/g or more. An upper limit of the maximum hygroscopic exothermic degree is not particularly limited, but is usually 0.060°C/g or less.

The artificial fibroin fiber may contain hydrophobic artificial fibroin and/or may contain artificial fibroin having an average hydropathy index (average HI) of more than 0 in order to exhibit sufficient hygroscopic exothermicity.

The artificial fibroin fiber according to the present embodiment preferably has excellent heat retention, and a heat retention index thereof determined according to the following formula B may be 0.20 or more. heat retention index = heat retention ratio (%) / basis weight of sample (g/m2)

Here, in the present specification, the heat retention ratio means a heat retention ratio measured by a dry contact method using a Thermolabo type II tester (under wind of 30 cm/sec), and is a value measured by a method described in Reference Example below.

The heat retention index of the artificial fibroin fiber according to the present embodiment may be 0.22 or more, 0.24 or more, 0.26 or more, 0.28 or more, 0.30 or more, or 0.32 or more. An upper limit of the heat retention index is not particularly limited, but may be, for example, 0.60 or less or 0.40 or less.

In the present specification, the "active component" means a component exhibiting a deodorant effect. The deodorant effect can be evaluated, for example, by a method described in SEK mark textile product authentication standard (JEC301, ISO17299). The SEK mark textile product authentication standard is applied to authentication for antibacterial deodorization processing, deodorization processing, antifouling processing, and the like, and is an authentication standard related to sales in China, Taiwan, Hong Kong, Indonesia, Turkey, Malaysia, Thailand, Vietnam, Singapore, India, South Korea, and the like. Examples of a test method include a detection tube method (ISO17299-2), a gas chromatography method (ISO17299-3A), and a sensory test method. In the detection tube method, the humidity of a sample having a specific sample size (100 cm² or 1.0 g) is controlled at 20°C and 65% RH for 24 hours or more, and then the sample is put into a 5 L-sized sampling bag. A measurement target gas adjusted to a predetermined concentration is injected thereinto. A gas concentration after a certain period of time is measured with a detection tube, and a weight reduction ratio thereof is calculated. Examples of the measurement target gas (odor component) include ammonia, acetic acid, methyl mercaptan, hydrogen sulfide, acetaldehyde, pyridine, and trimethylamine. In the gas chromatography method, the humidity of a sample having a specific sample size (50 cm² or 0.5 g) is controlled at 20°C and 65% RH for 24 hours or more, and then the sample is put into a 500 mL-sized Erlenmeyer flask. A measurement target solution adjusted to a predetermined concentration is added dropwise thereto. After a certain period of time, measurement is performed by gas chromatography, and a reduction ratio thereof is calculated. In the sensory test method, the humidity of a sample having a specific sample size (100 cm² or 1.0 g) is controlled at 20°C and 65% RH for 24 hours or more, and then the sample is put into a 500 mL-sized Erlenmeyer flask. An adjusted measurement target gas is injected into the Erlenmeyer flask, and the Erlenmeyer flask is sealed. After a certain period of time, six panelists determine whether the intensity of an atmospheric odor is stronger or weaker than a reference odor according to the following 6-stage odor intensity display method. An odor in the Erlenmeyer flask and an odor of a test piece after the test are compared with a determination odor (corresponding to odor intensity 2), and when five or more of the six panelists determine that both the odor in the flask and the odor of the test piece are equal to or less than the determination odor intensity, it is determined that there is a deodorant effect. For a woven fabric, a knit, a nonwoven fabric, a tape, and the like, an area is used as a standard, and for a yarn, a fiber, and the like, a weight is used as a standard. The definition of the odor intensity is as presented in Table 1.

**[Table 1]**

| Odor intensity | Contents |
|---|---|
| 0 | Odorless |
| 1 | Odor that is sensible with difficulty (detection threshold concentration) |
| 2 | Weak odor whose origin can be recognized (recognition threshold concentration) |
| 3 | Odor that is easily sensible |
| 4 | Strong odor |
| 5 | Overpowering odor |

The detection tube method and the gas chromatography method are referred to as an instrumental analysis test, and may be distinguished from the sensory test method. According to the SEK mark textile product authentication standard (ISO17299) of the Japan Textile Evaluation Council, in order to display an SEK Mark on an article, it is necessary to perform both the instrumental analysis test and the sensory test method, and to satisfy both the standards. Note that when a result of the instrumental analysis test satisfies a standard of a specific odor component reduction ratio, evaluation by the sensory test method can be omitted.

In the present specification, it can be determined that there is a deodorant effect when an odor component reduction ratio to an initial concentration (30 ppm) of acetic acid is 70% or more. Acetic acid is known as an odor component that causes a sweat odor, an aging odor, an excretion odor, or a tobacco odor. The deodorant effect on acetic acid is more preferably 75% or more, 80% or more, 85% or more, 90% or more, or 95% or more.

The odor component reduction ratio can be calculated by Formula C-1 in a case of the detection tube method, and can be calculated by Formula C-2 in a case of the gas chromatography method. odor component reduction ratio (%) = (average value s0 of empty test - average value sm of measurement / (average value s0 of empty test) × 100 odor component reduction ratio (%) = (average value s0 of empty test peak area - average value sm of test sample peak area) / (average value s0 of empty test peak area) × 100

In an embodiment, as for the deodorant effect, an odor component reduction ratio to an initial concentration (38 ppm) of isovaleric acid is preferably 85% or more. Isovaleric acid is known as an odor component that causes a sweat odor or an aging odor. The deodorant effect on isovaleric acid is more preferably 87% or more, 90% or more, 93% or more, or 95% or more.

In another embodiment, the deodorant effect is preferably 70% or less with respect to an initial concentration of ammonia (100 ppm). Ammonia is known as an odor component that causes a sweat odor, an aging odor, an excretion odor, a tobacco odor, a garbage odor, or an ammonia odor.

The deodorant material according to the present embodiment has a deodorant effect on acetic acid and isovaleric acid, and therefore exhibits a deodorant effect on a sweat odor, an aging odor, an excretion odor, and a tobacco odor. In an embodiment, the deodorant material exhibits a deodorant effect on a garbage odor.

The deodorant material according to the present embodiment may further contain an additive in addition to artificial fibroin. Examples of the additive include a stabilizer, a colorant, a fragrance, and a preservative.

### <Artificial fibroin>

The "artificial fibroin" according to the present embodiment means artificially produced fibroin (artificial fibroin). The artificial fibroin may be fibroin having an amino acid sequence different from or identical to an amino acid sequence of naturally derived fibroin.

The artificial fibroin may be a fibrous protein having a structure similar to that of naturally derived fibroin, or may be fibroin having a sequence similar to a repetitive sequence of naturally derived fibroin. The "sequence similar to a repetitive sequence of fibroin" may actually be a sequence of naturally derived fibroin, or may be a sequence similar thereto.

The "artificial fibroin" may be fibroin whose amino acid sequence is modified based on naturally derived fibroin (for example, fibroin whose amino acid sequence is modified by altering a cloned gene sequence of naturally derived fibroin) or fibroin obtained by artificially designing an amino acid sequence independently of naturally derived fibroin (for example, fibroin having a desired amino acid sequence by chemically synthesizing a nucleic acid encoding the designed amino acid sequence) as long as the artificial fibroin has an amino acid sequence specified in the present disclosure. Note that fibroin obtained by modifying an amino acid sequence of artificial fibroin is also included in artificial fibroin as long as the amino acid sequence thereof is different from the amino acid sequence of naturally derived fibroin. Examples of the artificial fibroin include artificial silk fibroin (fibroin obtained by modifying an amino acid sequence of a silk protein produced by silkworms) and artificial spider silk fibroin (fibroin obtained by modifying an amino acid sequence of a spider silk protein produced by spiders). When a synthetic polymer according to the present disclosure is used as a forming material, the artificial fibroin preferably contains artificial spider silk fibroin, and more preferably consists of artificial spider silk fibroin because the spider silk fibroin is relatively easily fibrillated and has a high fiber forming ability.

The artificial fibroin according to the present embodiment may be a protein having a domain sequence represented by Formula 1: [(A)ₙ motif-REP]ₘ or Formula 2: [(A)ₙ motif-REP]ₘ-(A)ₙ motif. An amino acid sequence (N-terminal sequence or C-terminal sequence) may be further added to any one or both of the N-terminal side and the C-terminal side of the domain sequence of the artificial fibroin. The N-terminal sequence and the C-terminal sequence are typically regions not containing repeats of amino acid motifs that are characteristic of fibroin and each consist of about 100 residues of amino acids, but are not limited thereto.

The term "domain sequence" in the present specification is an amino acid sequence that produces a crystal region (typically, corresponding to the (A)ₙ motif of the amino acid sequence) and an amorphous region (typically, corresponding to REP of the amino acid sequence) specific to fibroin, and means an amino acid sequence represented by Formula 1: [(A)ₙ motif-REP]ₘ or Formula 2: [(A)ₙ motif-REP]ₘ-(A)ₙ motif. Here, the (A)ₙ motif represents an amino acid sequence mainly consisting of alanine residues, and the number of amino acid residues is 2 to 27. The number of amino acid residues in the (A)ₙ motif may be an integer of 2 to 20, 4 to 27, 4 to 20, 8 to 20, 10 to 20, 4 to 16, 8 to 16, or 10 to 16. A ratio of the number of alanine residues to the total number of amino acid residues in the (A)ₙ motif only needs to be 40% or more, and may be 60% or more, 70% or more, 80% or more, 83% or more, 85% or more, 86% or more, 90% or more, 95% or more, or 100% (which means that the (A)ₙ motif consists only of alanine residues). At least seven of a plurality of (A)ₙ motifs in the domain sequence may consist only of alanine residues. The REP represents an amino acid sequence consisting of 2 to 200 amino acid residues. The REP may be an amino acid sequence consisting of 10 to 200 amino acid residues, or may be an amino acid sequence consisting of 10 to 40, 10 to 60, 10 to 80, 10 to 100, 10 to 120, 10 to 140, 10 to 160, or 10 to 180 amino acid residues, m represents an integer of 2 to 300, and may be an integer of 8 to 300, 10 to 300, 20 to 300, 40 to 300, 60 to 300, 80 to 300, 100 to 300, 10 to 200, 20 to 200, 20 to 180, 20 to 160, 20 to 140, or 20 to 120. The plurality of (A)ₙ motifs may be the same amino acid sequence or different amino acid sequences. The plurality of REPs may be the same amino acid sequence or different amino acid sequences.

The artificial fibroin according to the present embodiment can be obtained by, for example, performing modification of an amino acid sequence corresponding to substitution, deletion, insertion, and/or addition of one or a plurality of amino acid residues with respect to a cloned gene sequence of naturally derived fibroin. The substitution, deletion, insertion, and/or addition of amino acid residues can be performed by a method known to those skilled in the art, such as a site-directed mutagenesis method. Specifically, the substitution, deletion, insertion, and/or addition of amino acid residues can be performed according to methods recited in Nucleic Acid Res. 10, 6487 (1982), Methods in Enzymology, 100, 448 (1983), and the like.

The naturally derived fibroin is a protein having a domain sequence represented by Formula 1: [(A)ₙ motif-REP]ₘ or Formula 2: [(A)ₙ motif-REP]ₘ-(A)ₙ motif, and specific examples thereof include fibroin produced by insects or spiders.

Examples of the fibroin produced by insects include silk proteins produced by silkworms, such as Bombyx mori, Bombyx mandarina, Antheraea yamamai, Anteraea pernyi, Eriogyna pyretorum, Pilosamia Cynthia ricini, Samia cynthia, Caligura japonica, Antheraea mylitta, and Antheraea assama, and a hornet silk protein secreted by larvae of Vespa simillima xanthoptera.

More specific examples of the fibroin produced by insects include silkworm fibroin L chain (GenBank Accession Numbers. M76430 (base sequence) and AAA27840.1 (amino acid sequence)).

Examples of the fibroin produced by spiders include spider silk proteins produced by spiders belonging to the genus Araneus, such as Araneus ventricosus, Araneus diadematus, Araneus pinguis, Araneus pentagrammicus, and Araneus nojimai, spiders belonging to the genus Neoscona, such as Neoscona scylla, Neoscona nautica, Neoscona adianta, and Neoscona scylloides, spiders belonging to the genus Pronus, such as Pronous minutus, spiders belonging to the genus Cyrtarachne, such as Cyrtarachne bufo and Cyrtarachne inaequalis, spiders belonging to the genus Gasteracantha, such as Gasteracantha kuhlii and Gasteracantha mammosa, spiders belonging to the genus Ordgarius, such as Ordgarius hobsoni and Ordgarius sexspinosus, spiders belonging to the genus Argiope, such as Argiope amoena, Argiope minuta, and Argiope bruennichi, spiders belonging to the genus Arachnura, such as Arachnura logic, spiders belonging to the genus Acusilas, such as Acusilas coccineus, spiders belonging to the genus Cytophora, such as Cyrtophora moluccensis, Cyrtophora exanthematica, and Cyrtophora unicolor, spiders belonging to the genus Poltys, such as Poltys illepidus, spiders belonging to the genus Cyclosa, such as Cyclosa octotuberculata, Cyclosa sedeculata, Cyclosa vallata, and Cyclosa atrata, and spiders belonging to the genus Chorizopes, such as Chorizopes nipponicus, and spider silk proteins produced by spiders belonging to the family Tetragnathidae, such as spiders belonging to the genus Tetragnatha, such as Tetragnatha praedonia, Tetragnatha maxillosa, Tetragnatha extensa, and Tetragnatha squamata, spiders belonging to the genus Leucauge, such as Leucauge magnifica, Leucauge blanda, and Leucauge subblanda, spiders belonging to the genus Nephila, such as Nephila clavata and Nephila pilipes, spiders belonging to the genus Menosira, such as Menosira ornata, spiders belonging to the genus Dyschiriognatha, such as Dyschiriognatha tenera, spiders belonging to the genus Latrodectus, such as Latrodectus mactans, Latrodectus hasseltii, Latrodectus geometricus, and Latrodectus tredecimguttatus, and spiders belonging to the genus Euprosthenops. Examples of the spider silk protein include dragline silk proteins such as MaSps (MaSp1 and MaSp2) and ADFs (ADF3 and ADF4), and MiSps (MiSp1 and MiSp2).

More specific examples of the spider silk protein produced by spiders include fibroin-3 (adf-3) [derived from Araneus diadematus] (GenBank Accession No. AAC47010 (amino acid sequence), U47855 (base sequence)), fibroin-4 (adf-4) [derived from Araneus diadematus] (GenBank Accession No. AAC47011 (amino acid sequence), U47856 (base sequence)), dragline silk protein spidroin 1 [derived from Nephila clavipes] (GenBank Accession No. AAC04504 (amino acid sequence), U37520 (base sequence)), major ampullate spidroin 1 [derived from Latrodectus hesperus] (GenBank Accession No. ABR68856 (amino acid sequence), EF595246 (base sequence)), dragline silk protein spidroin 2 [derived from Nephila clavata] (GenBank Accession No. AAL32472 (amino acid sequence), AF441245 (base sequence)), major ampullate spidroin 1 [derived from Euprosthenops australis] (GenBank Accession No. CAJ00428 (amino acid sequence), AJ973155 (base sequence)), and major ampullate spidroin 2 [Euprosthenops australis] (GenBank Accession No. CAM32249.1 (amino acid sequence), AM490169 (base sequence)), minor ampullate silk protein 1 [Nephila clavipes] (GenBank Accession No. AAC14589.1 (amino acid sequence)), minor ampullate silk protein 2 [Nephila clavipes] (GenBank Accession No. AAC14591.1 (amino acid sequence)), and minor ampullate spidroin-like protein [Nephilengys cruentata] (GenBank Accession No. ABR37278.1 (amino acid sequence).

More specific examples of the naturally derived fibroin include fibroin having sequence information registered in NCBI GenBank. For example, it can be confirmed by extracting a sequence in which spidroin, ampullate, fibroin, "silk and polypeptide", or "silk and protein" is described as a keyword in DEFINITION from sequences including INV as DIVISION among pieces of sequence information registered in NCBI GenBank, a sequence in which a specific character string of product is described from CDS, or a sequence in which a character string specific to TISSUE TYPE is described from SOURCE.

The artificial fibroin according to the present embodiment may be artificial (modified) silk fibroin (obtained by modifying an amino acid sequence of a silk protein produced by silkworm), or may be artificial (modified) spider silk fibroin (obtained by modifying an amino acid sequence of a spider silk protein produced by spiders). As the artificial fibroin, artificial spider silk fibroin is preferable. The artificial spider silk fibroin is also excellent in heat retention, hygroscopic exothermicity, and/or flame retardancy.

Specific examples of the artificial fibroin include artificial fibroin derived from a major dragline silk protein produced in a major ampullate gland of a spider (first artificial fibroin), artificial fibroin containing a domain sequence in which the content of glycine residues is reduced (second artificial fibroin), artificial fibroin containing a domain sequence in which the content of an (A)ₙ motif is reduced (third artificial fibroin), artificial fibroin in which the content of glycine residues and the content of an (A)ₙ motif are reduced (fourth artificial fibroin), artificial fibroin containing a domain sequence including a region locally having a high hydropathy index (fifth artificial fibroin), and artificial fibroin containing a domain sequence in which the content of glutamine residues is reduced (sixth artificial fibroin).

Examples of the first artificial fibroin include a protein having a domain sequence represented by Formula 1: [(A)ₙ motif-REP]ₘ. In the first artificial fibroin, the number of amino acid residues in the (A)ₙ motif is preferably an integer of 3 to 20, more preferably an integer of 4 to 20, still more preferably an integer of 8 to 20, even still more preferably an integer of 10 to 20, still further preferably an integer of 4 to 16, particularly preferably an integer of 8 to 16, and most preferably an integer of 10 to 16. The number of amino acid residues constituting the REP in Formula 1 in the first artificial fibroin is preferably 10 to 200 residues, more preferably 10 to 150 residues, even more preferably 20 to 100 residues, and still more preferably 20 to 75 residues. In the first artificial fibroin, the total number of glycine residues, serine residues, and alanine residues included in the amino acid sequence represented by Formula 1: [(A)ₙ motif-REP]ₘ is preferably 40% or more, more preferably 60% or more, and still more preferably 70% or more, with respect to the total number of amino acid residues.

The first artificial fibroin may be a polypeptide having an amino acid sequence unit represented by Formula 1: [(A)ₙ motif-REP]ₘ, and having a C-terminal sequence which is an amino acid sequence set forth in any one of SEQ ID NOs: 1 to 3 or a C-terminal sequence which is an amino acid sequence having 90% or more homology with the amino acid sequence set forth in any one of SEQ ID NOs: 1 to 3.

The amino acid sequence set forth in SEQ ID NO: 1 is identical to an amino acid sequence consisting of 50 amino acid residues at the C-terminal of the amino acid sequence of ADF3 (GI: 1263287, NCBI), the amino acid sequence set forth in SEQ ID NO: 2 is identical to an amino acid sequence obtained by removing 20 amino acid residues from the C-terminal of the amino acid sequence set forth in SEQ ID NO: 1, and the amino acid sequence set forth in SEQ ID NO: 3 is identical to an amino acid sequence obtained by removing 29 residues from the C-terminal of the amino acid sequence set forth in SEQ ID NO: 1.

More specific examples of the first artificial fibroin include artificial fibroin having (1-i) an amino acid sequence set forth in SEQ ID NO: 4 (recombinant spider silk protein ADF3KaiLargeNRSH1) or (1-ii) an amino acid sequence having a sequence identity of 90% or more with the amino acid sequence set forth in SEQ ID NO: 4. The sequence identity is preferably 95% or more.

The amino acid sequence set forth in SEQ ID NO: 4 is obtained by the following mutation such that in the amino acid sequence of ADF3 having the N-terminal to which an amino acid sequence including a start codon, a His10 tag, and a human rhinovirus 3C protease (HRV3C protease) recognition site (SEQ ID NO: 5) are added, the first to thirteenth repetitive regions are roughly doubled and the translation ends at the 1,154th amino acid residue. The C-terminal amino acid sequence of the amino acid sequence set forth in SEQ ID NO: 4 is identical to the amino acid sequence set forth in SEQ ID NO: 3.

The artificial fibroin of (1-i) may consist of the amino acid sequence set forth in SEQ ID NO: 4.

The domain sequence of the second artificial fibroin has an amino acid sequence in which the content of glycine residues is reduced as compared with naturally derived fibroin. It can be said that the second artificial fibroin has an amino acid sequence corresponding to an amino acid sequence in which at least one or a plurality of glycine residues in REP are replaced with other amino acid residues as compared with naturally derived fibroin.

The domain sequence of the second artificial fibroin may have an amino acid sequence corresponding to an amino acid sequence in which one glycine residue in at least one or the plurality of motif sequences is replaced with another amino acid residue, in at least one motif sequence selected from GGX and GPGXX (where G represents a glycine residue, P represents a proline residue, and X represents an amino acid residue other than glycine) in the REP as compared with naturally derived fibroin.

In the second artificial fibroin, a ratio of the motif sequence described above in which a glycine residue is replaced with another amino acid residue to the total motif sequences may be 10% or more.

The second artificial fibroin contains a domain sequence represented by Formula 1: [(A)ₙ motif-REP]ₘ, and may have an amino acid sequence in which z/w is 30% or more, 40% or more, 50% or more, or 50.9% or more in a case where the total number of amino acid residues in the amino acid sequence consisting of XGX (where X represents an amino acid residue other than glycine) included in all REPs in a sequence excluding the sequence from the (A)ₙ motif located at the most C-terminal side to the C-terminal of the domain sequence from the domain sequence is defined as z, and the total number of amino acid residues in the sequence excluding the sequence from the (A)ₙ motif located at the most C-terminal side to the C-terminal of the domain sequence from the domain sequence is defined as w. The number of alanine residues with respect to the total number of amino acid residues in the (A)ₙ motif is 83% or more, preferably 86% or more, more preferably 90% or more, still more preferably 95% or more, and even still more preferably 100% (meaning that the (A)ₙ motif consists of only alanine residues).

The second artificial fibroin is preferably one in which a content ratio of an amino acid sequence consisting of XGX is increased by replacing one glycine residue of the GGX motif with another amino acid residue. A content ratio of the amino acid sequence consisting of GGX in the domain sequence of the second artificial fibroin is preferably 30% or less, more preferably 20% or less, even more preferably 10% or less, still more preferably 6% or less, still even more preferably 4% or less, and particularly preferably 2% or less. The content ratio of the amino acid sequence consisting of GGX in the domain sequence is calculated by a method similar to the following method for calculating a content ratio of the amino acid sequence consisting of XGX (z/w).

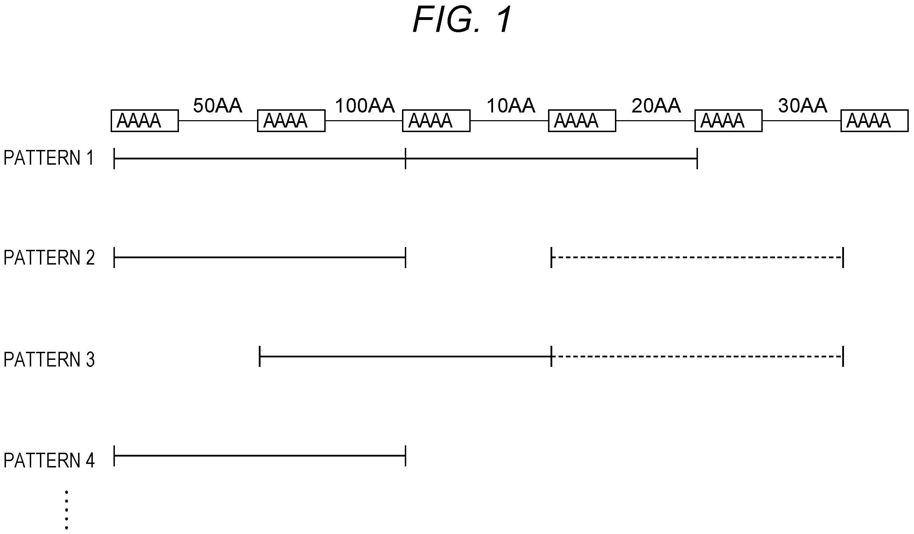

The method for calculating z/w will be described in more detail. First, the amino acid sequence consisting of XGX is extracted from all REP's included in a sequence excluding the sequence from the (A)ₙ motif located at the most C-terminal side to the C-terminal of the domain sequence from the domain sequence in the fibroin having a domain sequence represented by Formula 1: [(A)ₙ motif-REP]ₘ (artificial fibroin or naturally derived fibroin). The total number of amino acid residues included in XGX is represented by "z". For example, when 50 amino acid sequences consisting of XGX are extracted (no overlap), "z" is 50 × 3 = 150. In addition, for example, in an amino acid sequence consisting of XGXGX, one X (X in the center) is included in two XGXs, and the overlapping portion is subtracted to calculate "z" (that is, there are 5 amino acid residues in XGXGX). w is the total number of amino acid residues contained in a sequence excluding the sequence from the (A)ₙ motif located at the most C-terminal side to the C-terminal of the domain sequence from the domain sequence. For example, in the case of the domain sequence illustrated in Fig. 1, w is 4 + 50 + 4 + 100 + 4 + 10 + 4 + 20 + 4 + 30 = 230 (excluding the (A)ₙ motif located at the most C-terminal side). Next, z is divided by w to calculate z/w (%).

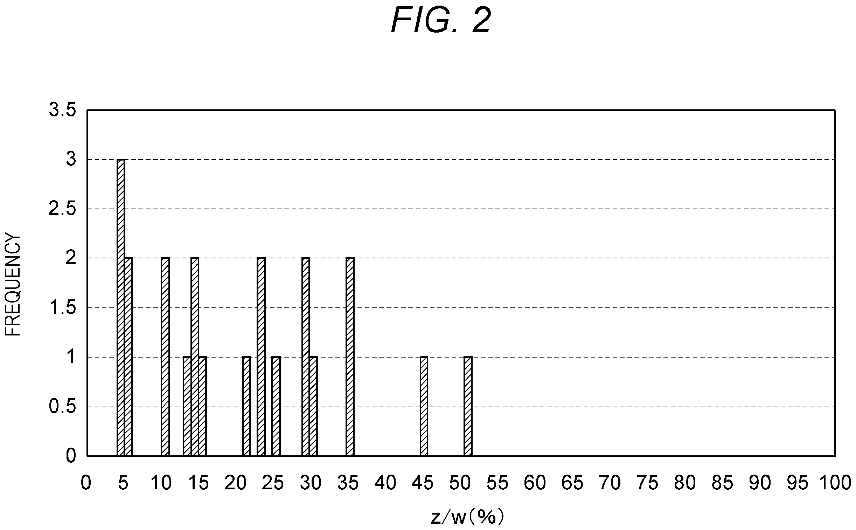

Here, z/w in the naturally derived fibroin will be described. First, fibroin whose amino acid sequence information was registered in NCBI GenBank was confirmed using the method exemplified above, and as a result, 663 types of fibroin (among these, 415 types were fibroin derived from spiders) were extracted. z/w was calculated by the calculation method described above from the amino acid sequences of the naturally derived fibroins which contain a domain sequence represented by Formula 1: [(A)ₙ motif-REP]ₘ and in which the content ratio of the amino acid sequence consisting of GGX in the fibroin is 6% or less, among all the extracted fibroins. Results thereof are illustrated in Fig. 2. In Fig. 2, the horizontal axis represents z/w (%), and the vertical axis represents a frequency. As is clear from Fig. 2, the values of z/w in the naturally derived fibroin are all lower than 50.9% (the largest value is 50.86%).

z/w in the second artificial fibroin is preferably 50.9% or more, more preferably 56.1% or more, even more preferably 58.7% or more, still more preferably 70% or more, and still even more preferably 80% or more. An upper limit of z/w is not particularly limited, and may be, for example, 95% or less.

The second artificial fibroin can be obtained by, for example, performing modification such that at least a part of base sequences encoding glycine residues in a cloned gene sequence for the naturally derived fibroin are replaced so as to encode another amino acid residue. In this case, one glycine residue in a GGX motif and GPGXX motif may be selected as a glycine residue to be modified or may be replaced such that z/w becomes 50.9% or more. It is also possible to obtain the second artificial fibroin by, for example, designing an amino acid sequence satisfying the above aspect from the amino acid sequence of the naturally derived fibroin and chemically synthesizing a nucleic acid encoding the designed amino acid sequence. In any case, in addition to the modification corresponding to a substitution of a glycine residue in the REP in the amino acid sequence of the naturally derived fibroin with another amino acid residue, further amino acid sequence modification may be performed, which corresponds to a substitution, a deletion, an insertion, and/or an addition of one or a plurality of amino acid residues.

Another amino acid residue above is not particularly limited as long as it is an amino acid residue other than a glycine residue, and the amino acid residue is preferably a hydrophobic amino acid residue such as a valine (V) residue, a leucine (L) residue, an isoleucine (I) residue, a methionine (M) residue, a proline (P) residue, a phenylalanine (F) residue, and a tryptophan (W) residue, and a hydrophilic amino acid residue such as a glutamine (Q) residue, an asparagine (N) residue, a serine (S) residue, a lysine (K) residue, and a glutamic acid (E) residue, more preferably a valine (V) residue, a leucine (L) residue, an isoleucine (I) residue, a phenylalanine (F) residue, and a glutamine (Q) residue, and even more preferably a glutamine (Q) residue.

More specific examples of the second artificial fibroin include artificial fibroin having (2-i) an amino acid sequence set forth in SEQ ID NO: 6 (Met-PRT380), SEQ ID NO: 7 (Met-PRT410), SEQ ID NO: 8 (Met-PRT525), or SEQ ID NO: 9 (Met-PRT799) or (2-ii) an amino acid sequence having a sequence identity of 90% or more with the amino acid sequence set forth in SEQ ID NO: 6, SEQ ID NO: 7, SEQ ID NO: 8, or SEQ ID NO: 9.

The artificial fibroin of (2-i) will be described. The amino acid sequence set forth in SEQ ID NO: 6 is obtained by replacing all GGX's in the REP in an amino acid sequence set forth in SEQ ID NO: 10 (Met-PRT313), which corresponds to the naturally derived fibroin, with GQX's. The amino acid sequence set forth in SEQ ID NO: 7 is obtained by deleting every other two (A)ₙ motifs from the N-terminal side to the C-terminal side from the amino acid sequence set forth in SEQ ID NO: 6 and further inserting one [(A)ₙ motif-REP] before the C-terminal sequence. The amino acid sequence set forth in SEQ ID NO: 8 is obtained by inserting two alanine residues on the C-terminal side of each (A)ₙ motif of the amino acid sequence set forth in SEQ ID NO: 7 and further replacing a part of glutamine (Q) residues with a serine (S) residue to delete a part of amino acids on the C-terminal side so as to be almost the same as the molecular weight of SEQ ID NO: 7. The amino acid sequence set forth in SEQ ID NO: 9 is obtained by adding a predetermined hinge sequence and a His tag sequence to the C-terminal of a sequence obtained by repeating a region of 20 domain sequences (where several amino acid residues on the C-terminal side of the region are substituted) present in the amino acid sequence set forth in SEQ ID NO: 7 four times.

A value of z/w in the amino acid sequence set forth in SEQ ID NO: 10 (corresponding to naturally derived fibroin) is 46.8%. Values of z/w in the amino acid sequence set forth in SEQ ID NO: 6, the amino acid sequence set forth in SEQ ID NO: 7, the amino acid sequence set forth in SEQ ID NO: 8, and the amino acid sequence set forth in SEQ ID NO: 9 are 58.7%, 70.1%, 66.1%, and 70.0%, respectively. Furthermore, values of x/y in the amino acid sequences set forth in SEQ ID NO: 10, SEQ ID NO: 6, SEQ ID NO: 7, SEQ ID NO: 8, and SEQ ID NO: 9 at a Giza ratio (to be described later) of 1:1.8 to 11.3 are 15.0%, 15.0%, 93.4%, 92.7%, and 89.8%, respectively.

The artificial fibroin of (2-i) may consist of the amino acid sequence set forth in SEQ ID NO: 6, SEQ ID NO: 7, SEQ ID NO: 8, or SEQ ID NO: 9.

The artificial fibroin of (2-ii) contains an amino acid sequence having a sequence identity of 90% or more with the amino acid sequence set forth in SEQ ID NO: 6, SEQ ID NO: 7, SEQ ID NO: 8, or SEQ ID NO: 9. The artificial fibroin of (2-ii) is also a protein having a domain sequence represented by Formula 1: [(A)ₙ motif-REP]ₘ. The sequence identity is preferably 95% or more.

It is preferable that the artificial fibroin of (2-ii) has 90% or more sequence identity with the amino acid sequence set forth in SEQ ID NO: 6, SEQ ID NO: 7, SEQ ID NO: 8, or SEQ ID NO: 9, and z/w is 50.9% or more in a case where the total number of amino acid residues in the amino acid sequence consisting of XGX (where X represents an amino acid residue other than glycine) included in the REP is defined as z, and the total number of amino acid residues of the REP in the domain sequence is defined as w.

The second artificial fibroin may have a tag sequence at one or both of the N-terminal and the C-terminal thereof. This makes it possible to isolate, immobilize, detect, and visualize the artificial fibroin.

Examples of the tag sequence include an affinity tag using specific affinity (binding properties or affinity) to another molecule. A specific example of the affinity tag includes a histidine tag (His tag). The His-tag is a short peptide in which about 4 to 10 histidine residues are lined up and can be used for isolating artificial fibroin by chelating metal chromatography, since it has a property of specifically binding to metal ions such as nickel. Specific examples of the tag sequence include an amino acid sequence set forth in SEQ ID NO: 11 (an amino acid sequence including a His-tag sequence and a hinge sequence).

In addition, it is possible to employ a tag sequence such as glutathione-S-transferase (GST) that specifically binds to glutathione or a maltose binding protein (MBP) that specifically binds to maltose.

An "epitope tag" utilizing an antigen-antibody reaction is employable. Adding a peptide (epitope) exhibiting antigenicity as a tag sequence allows binding of an antibody to the epitope. Examples of the epitope tag include an HA (a peptide sequence of influenza virus hemagglutinin) tag, a myc tag, a FLAG tag, and the like. The use of the epitope tag allows purification of the artificial fibroin to be easily performed with high specificity.

Furthermore, it is possible to use a tag sequence which is cleaved with a specific protease. By treating a protein adsorbed via the tag sequence with a protease, the artificial fibroin from which the tag sequence is cleaved can be recovered.

More specific examples of the artificial fibroin having a tag sequence include artificial fibroin having (2-iii) an amino acid sequence set forth in SEQ ID NO: 12 (PRT380), SEQ ID NO: 13 (PRT410), SEQ ID NO: 14 (PRT525), or SEQ ID NO: 15 (PRT799) or (2-iv) an amino acid sequence having a sequence identity of 90% or more with the amino acid sequence set forth in SEQ ID NO: 12, SEQ ID NO: 13, SEQ ID NO: 14, or SEQ ID NO: 15.

The amino acid sequences set forth in SEQ ID NO: 16 (PRT313), SEQ ID NO: 12, SEQ ID NO: 13, SEQ ID NO: 14, and SEQ ID NO: 15 are obtained by adding the amino acid sequence set forth in SEQ ID NO: 11 (which has a His-tag sequence and a hinge sequence) to the N-terminal of the amino acid sequences set forth in SEQ ID NO: 10, SEQ ID NO: 6, SEQ ID NO: 7, SEQ ID NO: 8, and SEQ ID NO: 9, respectively.

The artificial fibroin of (2-iii) may consist of the amino acid sequence set forth in SEQ ID NO: 12, SEQ ID NO: 13, SEQ ID NO: 14, or SEQ ID NO: 15.

The artificial fibroin of (2-iv) contains an amino acid sequence having a sequence identity of 90% or more with the amino acid sequence set forth in SEQ ID NO: 12, SEQ ID NO: 13, SEQ ID NO: 14, or SEQ ID NO: 15. The artificial fibroin of (2-iv) is also a protein having a domain sequence represented by Formula 1: [(A)ₙ motif-REP]ₘ. The sequence identity is preferably 95% or more.

It is preferable that the artificial fibroin of (2-iv) has 90% or more sequence identity with the amino acid sequence set forth in SEQ ID NO: 12, SEQ ID NO: 13, SEQ ID NO: 14, or SEQ ID NO: 15, and z/w is 50.9% or more in a case where the total number of amino acid residues in the amino acid sequence consisting of XGX (where X represents the amino acid residue other than glycine) in the REP is defined as z, and the total number of amino acid residues in the REP in the domain sequence is defined as w.

The second artificial fibroin may contain a secretory signal for releasing a protein produced in a recombinant protein production system to the outside of a host. A sequence of the secretory signal is designed appropriately depending on the type of the host.

The domain sequence of the third artificial fibroin has an amino acid sequence in which the content of the (A)ₙ motif is reduced, as compared with naturally derived fibroin. It can be said that the domain sequence of the third artificial fibroin has an amino acid sequence corresponding to an amino acid sequence in which at least one or a plurality of (A)ₙ motifs are deleted, as compared with naturally derived fibroin.

The third artificial fibroin may have an amino acid sequence corresponding to an amino acid sequence in which 10 to 40% of the (A)ₙ motifs are deleted from naturally derived fibroin.

The domain sequence of the third artificial fibroin may have an amino acid sequence corresponding to an amino acid sequence in which at least one (A)ₙ motif of every one to three (A)ₙ motifs is deleted from the N-terminal side to the C-terminal side, as compared with naturally derived fibroin.

The domain sequence of the third artificial fibroin may have an amino acid sequence corresponding to an amino acid sequence in which deletion of at least two consecutive (A)ₙ motifs and deletion of one (A)ₙ motif are repeated in this order from the N-terminal side to the C-terminal side, as compared with naturally derived fibroin.

The domain sequence of the third artificial fibroin may have an amino acid sequence corresponding to an amino acid sequence in which at least (A)ₙ motif every other two positions is deleted from the N-terminal side to the C-terminal side.

The third artificial fibroin contains a domain sequence represented by Formula 1: [(A)ₙ motif-REP]ₘ, and may have an amino acid sequence in which x/y is 20% or more, 30% or more, 40% or more, or 50% or more in a case where the numbers of amino acid residues in REPs of two adjacent [(A)ₙ motif-REP] units are sequentially compared from the N-terminal side to the C-terminal side, and the number of amino acid residues in one REP having a smaller number of amino acid residues is defined as 1, the maximum value of the total value of the number of amino acid residues in the two adjacent [(A)ₙ motif-REP] units where the ratio of the number of amino acid residues in the other REPs is 1.8 to 11.3 is defined as x, and the total number of amino acid residues in the domain sequence is defined as y. The number of alanine residues with respect to the total number of amino acid residues in the (A)ₙ motif is 83% or more, preferably 86% or more, more preferably 90% or more, still more preferably 95% or more, and even still more preferably 100% (meaning that the (A)ₙ motif consists of only alanine residues).

A method for calculating x/y will be described in more detail with reference to Fig. 1. Fig. 1 illustrates a domain sequence excluding the N-terminal sequence and the C-terminal sequence from the artificial fibroin. The domain sequence has a sequence of (A)ₙ motif-first REP (50 amino acid residues)-(A)ₙ motif-second REP (100 amino acid residues)-(A)ₙ motif-third REP (10 amino acid residues)-(A)ₙ motif-fourth REP (20 amino acid residues)-(A)ₙ motif-fifth REP (30 amino acid residues)-(A)ₙ motif from the N-terminal side (left side).

The two adjacent [(A)ₙ motif-REP] units are sequentially selected from the N-terminal side to the C-terminal side so as not to overlap. In this case, an unselected [(A)ₙ motif-REP] unit may exist. Fig. 1 illustrates a pattern 1 (a comparison between a first REP and a second REP and a comparison between a third REP and a fourth REP), a pattern 2 (a comparison between a first REP and a second REP and a comparison between a fourth REP and a fifth REP), a pattern 3 (a comparison between a second REP and a third REP and a comparison between a fourth REP and a fifth REP), and a pattern 4 (a comparison between a first REP and a second REP). Note that [(A)ₙ motif-REP] units may be selected in other ways.

Subsequently, the number of amino acid residues of each REP in the selected two adjacent [(A)ₙ motif-REP] units is compared for each pattern. The comparison is performed by setting the smaller number of amino acid residues as 1 and determining the ratio of the number of amino acid residues in the other REPs. For example, comparing the first REP (50 amino acid residues) and the second REP (100 amino acid residues), a ratio of amino acid residues in the second REP is 100/50 = 2, defining the first REP having fewer amino acid residues as 1. Similarly, comparing the fourth REP (20 amino acid residues) and the fifth REP (30 amino acid residues), a ratio of amino acid residues in the fifth REP is 30/20 = 1.5, defining the fourth REP having fewer amino acid residues as 1.

In Fig. 1, a set of [(A)ₙ motif-REP] units in which the ratio of the number of amino acid residues in the other REPs when one REP having a smaller number of amino acid residues is 1 is 1.8 to 11.3 is indicated by a solid line. In the present specification, the ratio is referred to as a Giza ratio. A set of [(A)ₙ motif-REP] units in which the ratio of the number of amino acid residues of the other REPs is less than 1.8 or more than 11.3 in a case where one REP having a smaller number of amino acid residues is defined as 1 is indicated by a broken line.

In each pattern, the number of all amino acid residues of two adjacent [(A)ₙ motif-REP] units indicated by solid lines (including not only the number of amino acid residues of the REP but also the number of amino acid residues of the (A)ₙ motif) is combined. The total values of the patterns are compared, and one that has the highest value (the maximum total value) is defined as x. In the example illustrated in Fig. 1, the total value of the pattern 1 is the maximum.

Then, x is divided by the total number of amino acid residues y of the domain sequence to calculate x/y (%).

x/y in the third artificial fibroin is preferably 50% or more, more preferably 60% or more, even more preferably 65% or more, still more preferably 70% or more, still even more preferably 75% or more, and particularly preferably 80% or more. An upper limit of x/y is not particularly limited, and may be, for example, 100% or less. With a Giza ratio of 1:1.9 to 11.3, x/y is preferably 89.6% or more. With a Giza ratio of 1:1.8 to 3.4, x/y is preferably 77.1% or more. When a Giza ratio is 1:1.9 to 8.4, x/y is preferably 75.9% or more. With a Giza ratio of 1:1.9 to 4.1, x/y is preferably 64.2% or more.

In a case where the third artificial fibroin is artificial fibroin in which at least seven of a plurality of (A)ₙ motifs in the domain sequence consist of only alanine residues, x/y is preferably 46.4% or more, more preferably 50% or more, still more preferably 55% or more, even still more preferably 60% or more, still further preferably 70% or more, and particularly preferably 80% or more. An upper limit of x/y is not particularly limited as long as the value is 100% or less.

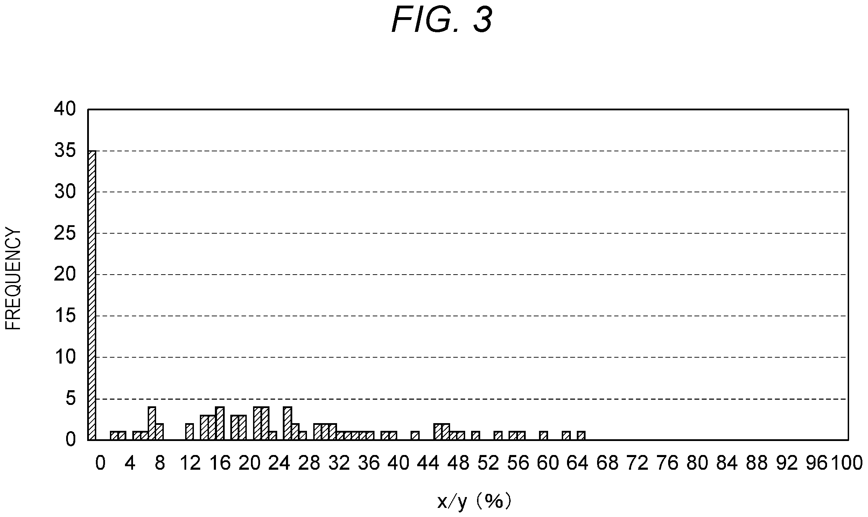

Here, x/y in the naturally derived fibroin will be described. First, fibroin whose amino acid sequence information was registered in NCBI GenBank was confirmed using the method exemplified above, and as a result, 663 types of fibroin (among these, 415 types were fibroin derived from spiders) were extracted. The values of x/y were calculated by the calculation method described above, from amino acid sequences of naturally derived fibroins consisting of a domain sequence represented by Formula 1: [(A)ₙ motif-REP]ₘ, among all the extracted fibroins. The results in a case where the Giza ratio is 1:1.9 to 4.1 are illustrated in Fig. 3.

In Fig. 3, the horizontal axis represents x/y (%), and the vertical axis represents a frequency. As is clear from Fig. 3, the values of x/y in the naturally derived fibroin are all lower than 64.2% (the largest value is 64.14%) .

The third artificial fibroin can be obtained from, for example, a cloned gene sequence of naturally derived fibroin, by deleting one or a plurality of sequences encoding an (A)ₙ motif such that x/y is 64.2% or more. In addition, for example, the third artificial fibroin can also be obtained, from the amino acid sequence of naturally derived fibroin, by designing an amino acid sequence corresponding to an amino acid sequence in which one or a plurality of (A)ₙ motifs are deleted such that x/y is 64.2% or more, and chemically synthesizing a nucleic acid encoding the designed amino acid sequence. In any case, in addition to the modification corresponding to deletion of the (A)ₙ motif from the amino acid sequence of naturally derived fibroin, modification of the amino acid sequence corresponding to substitution, deletion, insertion, and/or addition of one or a plurality of amino acid residues may be performed.

More specific examples of the third artificial fibroin include artificial fibroin having (3-i) an amino acid sequence set forth in SEQ ID NO: 17 (Met-PRT399), SEQ ID NO: 7 (Met-PRT410), SEQ ID NO: 8 (Met-PRT525), or SEQ ID NO: 9 (Met-PRT799) or (3-ii) an amino acid sequence having a sequence identity of 90% or more with the amino acid sequence set forth in SEQ ID NO: 17, SEQ ID NO: 7, SEQ ID NO: 8, or SEQ ID NO: 9.

The artificial fibroin of (3-i) will be described. The amino acid sequence set forth in SEQ ID NO: 17 is obtained by deleting every other two (A)ₙ motifs from the N-terminal side to the C-terminal side from the amino acid sequence set forth in SEQ ID NO: 10 (Met-PRT313) corresponding to naturally derived fibroin and further inserting one [(A)ₙ motif-REP] before the C-terminal sequence. The amino acid sequence set forth in SEQ ID NO: 7, SEQ ID NO: 8, or SEQ ID NO: 9 is as described in the second artificial fibroin.

A value of x/y in the amino acid sequence set forth in SEQ ID NO: 10 (corresponding to the naturally derived fibroin) at a Giza ratio of 1:1.8 to 11.3 is 15.0%. Values of x/y in the amino acid sequence set forth in SEQ ID NO: 17 and the amino acid sequence set forth in SEQ ID NO: 7 are both 93.4%. A value of x/y in the amino acid sequence set forth in SEQ ID NO: 8 is 92.7%. A value of x/y in the amino acid sequence set forth in SEQ ID NO: 9 is 89.8%. Values of z/w in the amino acid sequences set forth in SEQ ID NO: 10, SEQ ID NO: 17, SEQ ID NO: 7, SEQ ID NO: 8, and SEQ ID NO: 9 are 46.8%, 56.2%, 70.1%, 66.1%, and 70.0%, respectively.

The artificial fibroin of (3-i) may consist of the amino acid sequence set forth in SEQ ID NO: 17, SEQ ID NO: 7, SEQ ID NO: 8, or SEQ ID NO: 9.

The artificial fibroin of (3-ii) contains an amino acid sequence having a sequence identity of 90% or more with the amino acid sequence set forth in SEQ ID NO: 17, SEQ ID NO: 7, SEQ ID NO: 8, or SEQ ID NO: 9. The artificial fibroin of (3-ii) is also a protein having a domain sequence represented by Formula 1: [(A)ₙ motif-REP]ₘ. The sequence identity is preferably 95% or more.

It is preferable that the artificial fibroin of (3-ii) has 90% or more sequence identity with the amino acid sequence set forth in SEQ ID NO: 17, SEQ ID NO: 7, SEQ ID NO: 8, or SEQ ID NO: 9, and x/y is 64.2% or more in a case where the numbers of amino acid residues in REPs of two adjacent [(A)ₙ motif-REP] units are sequentially compared from the N-terminal side to the C-terminal side, and the number of amino acid residues in one REP having a small number of amino acid residues is defined as 1, the maximum value of the total value of the number of amino acid residues in the two adjacent [(A)ₙ motif-REP] units where the ratio of the number of amino acid residues in the other REPs is 1.8 to 11.3 (the Giza ratio is 1:1.8 to 11.3) is defined as x, and the total number of amino acid residues in the domain sequence is defined as y.

The third artificial fibroin may have the tag sequence described above at one or both of the N-terminal and the C-terminal thereof.

More specific examples of the artificial fibroin having a tag sequence include artificial fibroin having (3-iii) an amino acid sequence set forth in SEQ ID NO: 18 (PRT399), SEQ ID NO: 13 (PRT410), SEQ ID NO: 14 (PRT525), or SEQ ID NO: 15 (PRT799) or (3-iv) an amino acid sequence having a sequence identity of 90% or more with the amino acid sequence set forth in SEQ ID NO: 18, SEQ ID NO: 13, SEQ ID NO: 14, or SEQ ID NO: 15.

The amino acid sequences set forth in SEQ ID NO: 18, SEQ ID NO: 13, SEQ ID NO: 14, and SEQ ID NO: 15 are obtained by adding the amino acid sequence set forth in SEQ ID NO: 11 (which has a His-tag sequence and a hinge sequence) to the N-terminal of the amino acid sequences set forth in SEQ ID NO: 17, SEQ ID NO: 7, SEQ ID NO: 8, and SEQ ID NO: 9, respectively.

The artificial fibroin of (3-iii) may consist of the amino acid sequence set forth in SEQ ID NO: 18, SEQ ID NO: 13, SEQ ID NO: 14, or SEQ ID NO: 15.

The artificial fibroin of (3-iv) contains an amino acid sequence having a sequence identity of 90% or more with the amino acid sequence set forth in SEQ ID NO: 18, SEQ ID NO: 13, SEQ ID NO: 14, or SEQ ID NO: 15. The artificial fibroin of (3-iv) is also a protein having a domain sequence represented by Formula 1: [(A)ₙ motif-REP]ₘ. The sequence identity is preferably 95% or more.

It is preferable that the artificial fibroin of (3-iv) has 90% or more sequence identity with the amino acid sequence set forth in SEQ ID NO: 18, SEQ ID NO: 13, SEQ ID NO: 14, or SEQ ID NO: 15, and x/y is 64.2% or more in a case where the number of amino acid residues in REPs in two adjacent [(A)ₙ motif-REP] units are sequentially compared from the N-terminal side to the C-terminal side, and the number of amino acid residues in one REP having a small number of amino acid residues is defined as 1, the maximum value of the total value of the number of amino acid residues in the two adjacent [(A)ₙ motif-REP] units where the ratio of the number of amino acid residues in the other REPs is 1.8 to 11.3 is defined as x, and the total number of amino acid residues in the domain sequence is defined as Y.

The third artificial fibroin may contain a secretory signal for releasing a protein produced in a recombinant protein production system to the outside of a host. A sequence of the secretory signal is designed appropriately depending on the type of the host.

The domain sequence of the fourth artificial fibroin has an amino acid sequence in which the content of an (A)ₙ motif and the content of glycine residues are reduced, as compared with naturally derived fibroin. It can be said that the domain sequence of the fourth artificial fibroin has an amino acid sequence corresponding to an amino acid sequence in which at least one or a plurality of (A)ₙ motifs are deleted and at least one or a plurality of glycine residues in the REP are replaced with another amino acid residue, as compared with naturally derived fibroin. That is, the fourth artificial fibroin is artificial fibroin having characteristics of both the second artificial fibroin and the third artificial fibroin described above. Specific aspects thereof and the like are as in the descriptions for the second artificial fibroin and the third artificial fibroin.

More specific examples of the fourth artificial fibroin include artificial fibroin having (4-i) an amino acid sequence set forth in SEQ ID NO: 7 (Met-PRT410), SEQ ID NO: 8 (Met-PRT525), SEQ ID NO: 9 (Met-PRT799), SEQ ID NO: 13 (PRT410), SEQ ID NO: 14 (PRT525), or SEQ ID NO: 15 (PRT799) or (4-ii) an amino acid sequence having a sequence identity of 90% or more with the amino acid sequence set forth in SEQ ID NO: 7, SEQ ID NO: 8, SEQ ID NO: 9, SEQ ID NO: 13, SEQ ID NO: 14, or SEQ ID NO: 15. Specific aspects of the artificial fibroin containing the amino acid sequence set forth in SEQ ID NO: 7, SEQ ID NO: 8, SEQ ID NO: 9, SEQ ID NO: 13, SEQ ID NO: 14, or SEQ ID NO: 15 are as described above.

The domain sequence of the fifth artificial fibroin may have an amino acid sequence containing a region in which a hydropathy index is locally high, which corresponds to an amino acid sequence in which one or a plurality of amino acid residues in the REP are replaced with amino acid residues having a high hydropathy index, and/or an amino acid sequence in which one or a plurality of amino acid residues having a high hydropathy index are inserted into REP as compared with the naturally derived fibroin.

The region locally having a high hydropathy index preferably includes two to four consecutive amino acid residues.

The amino acid residue having a high hydropathy index is more preferably an amino acid residue selected from isoleucine (I), valine (V), leucine (L), phenylalanine (F), cysteine (C), methionine (M), and alanine (A).

In addition to the modification corresponding to a substitution of one or a plurality of amino acid residues in the REP with amino acid residues having a high hydropathy index and/or an insertion of one or a plurality of amino acid residues having a high hydropathy index into REP as compared with the naturally derived fibroin, further amino acid sequence modification may be performed on the fifth artificial fibroin, which corresponds to a substitution, a deletion, an insertion, and/or an addition of one or a plurality of amino acid residues as compared with the naturally derived fibroin.

The fifth artificial fibroin can be obtained from, for example, a cloned gene sequence for the naturally derived fibroin by replacing one or a plurality of hydrophilic amino acid residues (for example, amino acid residues having a negative value of hydropathy index) in the REP with hydrophobic amino acid residues (for example, amino acid residues having a positive value of hydropathy index) and/or by inserting one or a plurality of hydrophobic amino acid residues into REP. It is also possible to obtain the fifth artificial fibroin by, for example, designing an amino acid sequence corresponding to an amino acid sequence in which one or a plurality of hydrophilic amino acid residues in REP in the amino acid sequence of the naturally derived fibroin are replaced with hydrophobic amino acid residues and/or an amino acid sequence in which one or a plurality of hydrophobic amino acid residues are inserted into REP in the amino acid sequence of the naturally derived fibroin and chemically synthesizing a nucleic acid encoding the designed amino acid sequence. In any case, in addition to the modification corresponding to a substitution of one or a plurality of hydrophilic amino acid residues in the REP in the amino acid sequence of the naturally derived fibroin with hydrophobic amino acid residues and/or an insertion of one or a plurality of hydrophobic amino acid residues into REP in the amino acid sequence of the naturally derived fibroin, further amino acid sequence modification may be performed, which corresponds to a substitution, a deletion, an insertion, and/or an addition of one or a plurality of amino acid residues.

The fifth artificial fibroin contains a domain sequence represented by Formula 1: [(A)ₙ motif-REP]ₘ, and may have an amino acid sequence in which p/q is 6.2% or more in a case where in all REP's included in a sequence excluding the sequence from the (A)ₙ motif located at the most C-terminal side to the C-terminal of the domain sequence from the domain sequence, the total number of amino acid residues included in a region where the average hydropathy index of four consecutive amino acid residues is 2.6 or more is defined as p, and the total number of amino acid residues included in the sequence excluding the sequence from the (A) ₙ motif located at the most C-terminal side to the C-terminal of the domain sequence from the domain sequence is defined as q.

As the hydropathy index of an amino acid residue, a known index is used herein (Hydropathy index: Kyte J, & Doolittle R (1982)" A simple method for displaying the hydropathic character of a protein", J. Mol. Biol., 157, pp. 105-132). Specifically, the hydropathy index (hereinafter, also referred to as "HI") of each amino acid is as shown in Table 2 below.

**[Table 2]**

| Amino acid | | HI | Amino acid | | HI |
|---|---|---|---|---|---|
| Isoleucine | Ile | 4.5 | Tryptophan | Trp | -0.9 |
| Valine | Val | 4.2 | Tyrosine | Tyr | -1.3 |
| Leucine | Leu | 3.8 | Proline | Pro | -1.6 |
| Phenylalanine | Phe | 2.8 | Histidine | His | -3.2 |
| Cysteine | Cys | 2.5 | Asparagine | Asn | -3.5 |
| Methionine | Met | 1.9 | Aspartic acid | Asp | -3.5 |
| Alanine | Ala | 1.8 | Glutamine | Gln | -3.5 |
| Glycine | Gly | -0.4 | Glutamic acid | glu | -3.5 |
| Threonine | Thr | -0.7 | Lysine | Lys | -3.9 |
| Serine | Ser | -0.8 | Arginine | Arg | -4.5 |

A method for calculating p/q will be described in more detail. In the calculation, a sequence excluding the sequence from the (A) ₙ motif located at the most C-terminal side to the C-terminal of the domain sequence from the domain sequence represented by Formula 1 [(A)ₙ motif-REP]ₘ (hereinafter also referred to as "sequence A") is used. First, in all REP's included in the sequence A, an average hydropathy index of four consecutive amino acid residues is calculated. An average hydropathy index is determined by dividing a sum of His of amino acid residues in four consecutive amino acid residues by 4 (the number of amino acid residues). An average hydropathy index is determined for all four consecutive amino acid residues (each amino acid residue is used for calculating an average one to four times). Then, a region where four consecutive amino acid residues have an average hydropathy index of 2.6 or more is determined. Even when a certain amino acid residue corresponds to a plurality of "four consecutive amino acid residues having an average hydropathy index of 2.6 or more", the region is regarded as including one amino acid residue. The total number of amino acid residues included in the region is "p". The total number of amino acid residues included in the sequence A is "q".

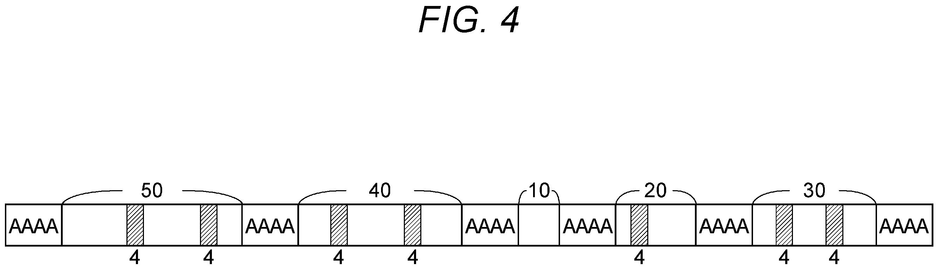

For example, in a case where "four consecutive amino acid residues having an average hydropathy index of 2.6 or more" are extracted from 20 places (no overlap), there are 20 sets of four consecutive amino acid residues (no overlap) in a region where four consecutive amino acid residues have an average hydropathy index of 2.6 or more, and thus, "p" is 20 × 4 = 80. In addition, for example, in a case where two of the "four consecutive amino acid residues having an average hydropathy index of 2.6 or more" overlap by only one amino acid residue, in the region where the average hydropathy index of four consecutive amino acid residues is 2.6 or more, the number of amino acid residues is 7 (p = 2 × 4 - 1 = 7. "-1" is a deduction of overlapping amino acid residue). For example, in the case of the domain sequence illustrated in Fig. 4, there are seven "four consecutive amino acid residues having an average hydropathy index of 2.6 or more" without overlapping, and thus p is 7 × 4 = 28. Furthermore, for example, in the case of the domain sequence shown in Fig. 4, q is 4 + 50 + 4 + 40 + 4 + 10 + 4 + 20 + 4 + 30 = 170 (the (A) ₙ motif located at the end of the C-terminal side is excluded). Next, p/q (%) can be calculated by dividing p by q. In the case of Fig. 4, p/q is 28/170 = 16.47%.

p/q in the fifth artificial fibroin is preferably 6.2% or more, more preferably 7% or more, even more preferably 10% or more, still more preferably 20% or more, and still even more preferably 30% or more. An upper limit of p/q is not particularly limited, and may be, for example, 45% or less.

The fifth artificial fibroin can be obtained by, for example, modifying a cloned amino acid sequence of the naturally derived fibroin into an amino acid sequence containing a region in which a hydropathy index is locally high by replacing one or a plurality of hydrophilic amino acid residues (for example, amino acid residues having a negative value of hydropathy index) in the REP with hydrophobic amino acid residues (for example, amino acid residues having a positive value of hydropathy index) and/or by inserting one or a plurality of hydrophobic amino acid residues into REP, such that the condition of p/q is satisfied. It is also possible to obtain the fifth artificial fibroin by, for example, designing an amino acid sequence satisfying the condition of p/q from the amino acid sequence of the naturally derived fibroin and chemically synthesizing a nucleic acid encoding the designed amino acid sequence. In any case, in addition to the modification corresponding to a substitution of one or a plurality of amino acid residues in the REP with amino acid residues having a high hydropathy index and/or an insertion of one or a plurality of amino acid residues having a high hydropathy index into REP as compared with the naturally derived fibroin, further modification may be performed, which corresponds to a substitution, a deletion, an insertion, and/or an addition of one or a plurality of amino acid residues.

The amino acid residue having a high hydropathy index is not particularly limited but is preferably isoleucine (I), valine (V), leucine (L), phenylalanine (F), cysteine (C), methionine (M), and alanine (A). Among these examples, valine (V), leucine (L), and isoleucine (I) are more preferable.

More specific examples of the fifth artificial fibroin include artificial fibroin having (5-i) an amino acid sequence set forth in SEQ ID NO: 19 (Met-PRT720), SEQ ID NO: 20 (Met-PRT665), or SEQ ID NO: 21 (Met-PRT666) or (5-ii) an amino acid sequence having a sequence identity of 90% or more with the amino acid sequence set forth in SEQ ID NO: 19, SEQ ID NO: 20, or SEQ ID NO: 21.

The artificial fibroin of (5-i) will be described. The amino acid sequence set forth in SEQ ID NO: 19 is obtained by inserting amino acid sequences consisting of three amino acid residues (VLI) at two sites for each REP in the amino acid sequence set forth in SEQ ID NO: 7 (Met-PRT410) excluding the terminal domain sequence on the C-terminal side, replacing a part of glutamine (Q) residues with serine (S) residues, and deleting a part of amino acids on the C-terminal side. The amino acid sequence set forth in SEQ ID NO: 20 is obtained by inserting an amino acid sequence consisting of three amino acid residues (VLI) at one site for each REP in the amino acid sequence set forth in SEQ ID NO: 8 (Met-PRT525). The amino acid sequence set forth in SEQ ID NO: 21 is obtained by inserting amino acid sequences consisting of three amino acid residues (VLI) at two sites for each REP in the amino acid sequence set forth in SEQ ID NO: 8.

The artificial fibroin of (5-i) may consist of the amino acid sequence set forth in SEQ ID NO: 19, SEQ ID NO: 20, or SEQ ID NO: 21.

The artificial fibroin of (5-ii) contains an amino acid sequence having a sequence identity of 90% or more with the amino acid sequence set forth in SEQ ID NO: 19, SEQ ID NO: 20, or SEQ ID NO: 21. The artificial fibroin of (5-ii) is also a protein having a domain sequence represented by Formula 1: [(A)ₙ motif-REP]ₘ. The sequence identity is preferably 95% or more.

It is preferable that the artificial fibroin of (5-ii) has 90% or more sequence identity with the amino acid sequence set forth in SEQ ID NO: 19, SEQ ID NO: 20, or SEQ ID NO: 21, and p/q is 6.2% or more in a case where in all REP's included in a sequence excluding the sequence from the (A) ₙ motif located at the most C-terminal side to the C-terminal of the domain sequence from the domain sequence, the total number of amino acid residues included in a region where the average hydropathy index of four consecutive amino acid residues is 2.6 or more is defined as p, and the total number of amino acid residues included in the sequence excluding the sequence from the (A)ₙ motif located at the most the C-terminal side to the C-terminal of the domain sequence from the domain sequence is defined as q.

The fifth artificial fibroin may have a tag sequence at one or both of the N-terminal and the C-terminal thereof.

More specific examples of the artificial fibroin having a tag sequence include artificial fibroin having (5-iii) an amino acid sequence set forth in SEQ ID NO: 22 (PRT720), SEQ ID NO: 23 (PRT665), or SEQ ID NO: 24 (PRT666) or (5-iv) an amino acid sequence having a sequence identity of 90% or more with the amino acid sequence set forth in SEQ ID NO: 22, SEQ ID NO: 23, or SEQ ID NO: 24.

The amino acid sequences set forth in SEQ ID NO: 22, SEQ ID NO: 23, and SEQ ID NO: 24 are obtained by adding the amino acid sequence set forth in SEQ ID NO: 11 (which has a His-tag sequence and a hinge sequence) to the N-terminal of the amino acid sequences set forth in SEQ ID NO: 19, SEQ ID NO: 20, and SEQ ID NO: 21, respectively.

The artificial fibroin of (5-iii) may consist of the amino acid sequence set forth in SEQ ID NO: 22, SEQ ID NO: 23, or SEQ ID NO: 24.

The artificial fibroin of (5-iv) contains an amino acid sequence having a sequence identity of 90% or more with the amino acid sequence set forth in SEQ ID NO: 22, SEQ ID NO: 23, or SEQ ID NO: 24. The artificial fibroin of (5-iv) is also a protein having a domain sequence represented by Formula 1: [(A)ₙ motif-REP]ₘ. The sequence identity is preferably 95% or more.

It is preferable that the artificial fibroin of (5-iv) has 90% or more sequence identity with the amino acid sequence set forth in SEQ ID NO: 22, SEQ ID NO: 23, or SEQ ID NO: 24, and p/q is 6.2% or more in a case where in all REP's included in a sequence excluding the sequence from the (A) ₙ motif located at the most C-terminal side to the C-terminal of the domain sequence from the domain sequence, the total number of amino acid residues included in a region where the average hydropathy index of four consecutive amino acid residues is 2.6 or more is defined as p, and the total number of amino acid residues included in the sequence excluding the sequence from the (A)ₙ motif located at the most the C-terminal side to the C-terminal of the domain sequence from the domain sequence is defined as q.

The fifth artificial fibroin may contain a secretory signal for releasing a protein produced in a recombinant protein production system to the outside of a host. A sequence of the secretory signal is designed appropriately depending on the type of the host.

The sixth artificial fibroin has an amino acid sequence in which a content of glutamine residues is reduced as compared with the naturally derived fibroin.

It is preferable that the sixth artificial fibroin contains at least one motif selected from a GGX motif and a GPGXX motif in the amino acid sequence of REP.

In a case where the sixth artificial fibroin contains the GPGXX motif in the REP, a content ratio of the GPGXX motifs is generally 1% or more. The content ratio of the GPGXX motif may be 5% or more and is preferably 10% or more. An upper limit of a content ratio of the GPGXX motif is not particularly limited and may be 50% or less or 30% or less.

In the present specification, the "content ratio of the GPGXX motif" is a value calculated by the following method.

The content ratio of the GPGXX motif in fibroin (artificial fibroin or naturally derived fibroin) having a domain sequence represented by Formula 1: [(A)ₙ motif-REP]ₘ or Formula 2: [(A)ₙ motif-REP]ₘ-(A)ₙ motif is calculated as s/t, in a case where the number obtained by tripling the total number of GPGXX motifs in regions of all REP's included in a sequence excluding the sequence from the (A)ₙ motif located at the most C-terminal side to the C-terminal of the domain sequence from the domain sequence (that is, corresponding to the total number of G and P in the GPGXX motifs) is defined as s, and the total number of amino acid residues in all REP's excluding a sequence from the (A)ₙ motif located at the most C-terminal side to the C-terminal of the domain sequence from the domain sequence and further excluding the (A)ₙ motifs is defined as t.

In the calculation of the content ratio of the GPGXX motif, the "sequence excluding the sequence from the (A)ₙ motif located at the most C-terminal side to the C-terminal of the domain sequence from the domain sequence" is used to exclude the effect occurring due to the fact that the "sequence from the (A) ₙ motif located at the most C-terminal side to the C-terminal of the domain sequence" (a sequence corresponding to REP) may include a sequence having a low correlation with the sequence characteristic of fibroin, which influences the calculation result of the content ratio of the GPGXX motif in a case where m is small (that is, in a case where the domain sequence is short). In a case where the "GPGXX motif" is located at the C-terminal of an REP, even when "XX" is "AA", for example, it is regarded as the "GPGXX motif".

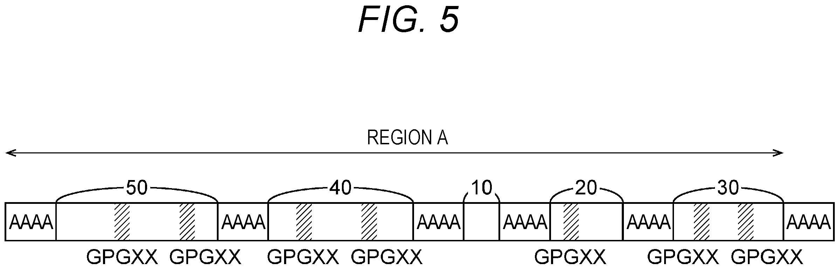

Fig. 5 is a schematic diagram illustrating a domain sequence of artificial fibroin. The calculation method of the content ratio of the GPGXX motif will be specifically described with reference to Fig. 5. First, in the domain sequence of the artificial fibroin ("[(A)ₙ motif-REP]ₘ-(A)ₙ motif" type) illustrated in Fig. 5 since all REP's are contained in the "sequence excluding the sequence from the (A) ₙ motif located at the most C-terminal side to the C-terminal of the domain sequence from the domain sequence" (the sequence indicated by the "region A" in Fig. 5), the number of GPGXX motifs for calculating s is 7, and s is 7 × 3 = 21. Similarly, since all REP's are included in the "sequence excluding the sequence from the (A)ₙ motif located at the most C-terminal side to the C-terminal of the domain sequence from the domain sequence" (in Fig. 5, the sequence indicated as the "region A"), the total number t of the amino acid residues in all REP's when the (A)ₙ motifs are further excluded from the sequence is 50 + 40 + 10 + 20 + 30 = 150. Next, s/t (%) can be calculated by dividing s by t, and in the case of the artificial fibroin of Fig. 5, s/t (%) is 21/150 = 14.0%.

The content ratio of glutamine residues in the sixth artificial fibroin is preferably 9% or less, more preferably 7% or less, even more preferably 4% or less, and particularly preferably 0%.

Herein, the "content ratio of glutamine residues" is a value calculated by the following method.

In fibroin (artificial fibroin or naturally derived fibroin) having a domain sequence represented by Formula 1: [(A)ₙ motif-REP]ₘ or Formula 2: [(A)ₙ motif-REP]ₘ-(A)ₙ motif, the content ratio of the glutamine residues is calculated as u/t, in which a total number of glutamine residues in regions of all REP's contained in a sequence excluding the sequence from the (A) ₙ motif located at the most C-terminal side to the C-terminal of the domain sequence from the domain sequence (sequence corresponding to the "region A" in Fig. 5) is u, and a total number of amino acid residues in all REP's excluding the sequence from the (A) ₙ motif located at the most C-terminal side to the C-terminal of the domain sequence from the domain sequence and further excluding (A) ₙ motifs is t. In the calculation of the content ratio of the glutamine residues, the reason for targeting the "sequence excluding the sequence from the (A) ₙ motif located at the most C-terminal side to the C-terminal of the domain sequence from the domain sequence" is the same as the reason described above.

The domain sequence of the sixth artificial fibroin may have an amino acid sequence corresponding to an amino acid sequence in which one or a plurality of glutamine residues in the REP are deleted or replaced with other amino acid residues, as compared with naturally derived fibroin.

As the "other amino acid residues", any amino acid residue other than the glutamine residue can be used, but an amino acid residue having a higher hydropathy index than the glutamine residue is preferable. The hydropathy index of the amino acid residue is as shown in Table 2.

As shown in Table 2, examples of the amino acid residue with a higher hydropathy index than that of the glutamine residue include amino acid residues selected from isoleucine (I), valine (V), leucine (L), phenylalanine (F), cysteine (C), methionine (M), alanine (A), glycine (G), threonine (T), serine (S), tryptophan (W), tyrosine (Y), proline (P), and histidine (H). Among these examples, the amino acid residue is more preferably one selected from isoleucine (I), valine (V), leucine (L), phenylalanine (F), cysteine (C), methionine (M), and alanine (A), and still more preferably one selected from isoleucine (I), valine (V), leucine (L), and phenylalanine (F).

In the sixth artificial fibroin, hydrophobicity of REP is preferably -0.8 or higher, more preferably -0.7 or higher, even more preferably 0 or higher, still more preferably 0.3 or higher, and particularly preferably 0.4 or higher. An upper limit of the hydrophobic degree of each REP is not particularly limited, and may be 1.0 or less or 0.7 or less.

Herein, the "hydrophobic degree of REP" is a value calculated by the following method.

In fibroin (artificial fibroin or naturally derived fibroin) having a domain sequence represented by Formula 1: [(A)ₙ motif-REP]ₘ or Formula 2: [(A)ₙ motif-REP]ₘ-(A)ₙ motif, the hydrophobic degree of REP is calculated as v/t, in which a sum of hydropathy indices of amino acid residues in regions of all REP's contained in the sequence excluding the sequence from the (A) ₙ motif located at the most C-terminal side to the C-terminal of the domain sequence from the domain sequence (sequence corresponding to the "region A" in Fig. 5) is v, and the total number of amino acid residues in all REP's excluding the sequence from the (A)ₙ motif located at the most C-terminal side to the C-terminal of the domain sequence from the domain sequence and further excluding (A) ₙ motifs is t. In the calculation of the hydrophobic degree of REP, the reason for targeting the "sequence excluding the sequence from the (A)ₙ motif located at the most C-terminal side to the C-terminal of the domain sequence from the domain sequence" is the same as the reason described above.

In addition to the modification corresponding to a deletion of one or a plurality of glutamine residues in the REP and/or a substitution of one or a plurality of glutamine residues in the REP with other amino acid residues as compared with the naturally derived fibroin, further amino acid sequence modification may be performed on the domain sequence of the sixth artificial fibroin, which corresponds to a substitution, a deletion, an insertion, and/or an addition of one or a plurality of amino acid residues.

The sixth artificial fibroin can be obtained from, for example, a cloned gene sequence for the naturally derived fibroin by deleting one or a plurality of glutamine residues in the REP and/or replacing one or a plurality of glutamine residues in the REP with other amino acid residues. It is also possible to obtain the sixth artificial fibroin by, for example, designing an amino acid sequence corresponding to an amino acid sequence in which one or a plurality of glutamine residues in REP in the amino acid sequence of the naturally derived fibroin are deleted and/or one or a plurality of glutamine residues in REP in the amino acid sequence of the naturally derived fibroin are replaced with other amino acid residues and chemically synthesizing a nucleic acid encoding the designed amino acid sequence.

More specific examples of the sixth artificial fibroin include artificial fibroin having (6-i) an amino acid sequence set forth in SEQ ID NO: 25 (Met-PRT888), SEQ ID NO: 26 (Met-PRT965), SEQ ID NO: 27 (Met-PRT889), SEQ ID NO: 28 (Met-PRT916), SEQ ID NO: 29 (Met-PRT918), SEQ ID NO: 30 (Met-PRT699), SEQ ID NO: 31 (Met-PRT698), SEQ ID NO: 32 (Met-PRT966), SEQ ID NO: 41 (Met-PRT917), or SEQ ID NO: 42 (Met-PRT1028) or artificial fibroin having (6-ii) an amino acid sequence having a sequence identity of 90% or more with the amino acid sequence set forth in SEQ ID NO: 25, SEQ ID NO: 26, SEQ ID NO: 27, SEQ ID NO: 28, SEQ ID NO: 29, SEQ ID NO: 30, SEQ ID NO: 31, SEQ ID NO: 32, SEQ ID NO: 41, or SEQ ID NO: 42.

The artificial fibroin of (6-i) will be described. The amino acid sequence set forth in SEQ ID NO: 25 is obtained by replacing all QQ's in the amino acid sequence set forth in SEQ ID NO: 7 (Met-PRT410) with VL's. The amino acid sequence set forth in SEQ ID NO: 26 is obtained by replacing all QQ's in the amino acid sequence set forth in SEQ ID NO: 7 with TS's and replacing the remaining Q's with A's. The amino acid sequence set forth in SEQ ID NO: 27 is obtained by replacing all QQ's in the amino acid sequence set forth in SEQ ID NO: 7 with VL's and replacing the remaining Q's with I's. The amino acid sequence set forth in SEQ ID NO: 28 is obtained by replacing all QQ's in the amino acid sequence set forth in SEQ ID NO: 7 with VI's and replacing the remaining Q's with L's. The amino acid sequence set forth in SEQ ID NO: 29 is obtained by replacing all QQ's in the amino acid sequence set forth in SEQ ID NO: 7 with VF's and replacing the remaining Q's with I's.

The amino acid sequence set forth in SEQ ID NO: 30 is obtained by replacing all QQ's in the amino acid sequence set forth in SEQ ID NO: 8 (Met-PRT525) with VL's. The amino acid sequence set forth in SEQ ID NO: 31 is obtained by replacing all QQ's in the amino acid sequence set forth in SEQ ID NO: 8 with VL's and replacing the remaining Q's with I's.

The amino acid sequence set forth in SEQ ID NO: 32 is obtained by replacing all QQ's with VF's in a sequence in which a region of 20 domain sequences existing in the amino acid sequence set forth in SEQ ID NO: 7 (Met-PRT410) is repeated twice and replacing the remaining Q's with I's.

The amino acid sequence set forth in SEQ ID NO: 41 (Met-PRT917) is obtained by replacing all QQ's in the amino acid sequence set forth in SEQ ID NO: 7 with LI's and replacing the remaining Q's with V's. The amino acid sequence set forth in SEQ ID NO: 42 (Met-PRT1028) is obtained by replacing all QQ's in the amino acid sequence set forth in SEQ ID NO: 7 with IF's and replacing the remaining Q's with T's.

The content ratio of the glutamine residues in each of the amino acid sequences set forth in SEQ ID NO: 25, SEQ ID NO: 26, SEQ ID NO: 27, SEQ ID NO: 28, SEQ ID NO: 29, SEQ ID NO: 30, SEQ ID NO: 31, SEQ ID NO: 32, SEQ ID NO: 41, and SEQ ID NO: 42 is 9% or less (Table 3).

**[Table 3]**

| Modified fibroin | | Content ratio of glutamine residues | Content ratio of GPGXX motif | Hydrophobic degree of REP |
|---|---|---|---|---|
| Met-PRT410 | SEQ ID NO: 7 | 17.7% | 27.9% | -1.52 |
| Met-PRT888 | SEQ ID NO: 25 | 6.3% | 27.9% | -0.07 |
| Met-PRT965 | SEQ ID NO: 26 | 0.0% | 27.9% | -0.65 |
| Met-PRT889 | SEQ ID NO: 27 | 0.0% | 27.9% | 0.35 |
| Met-PRT916 | SEQ ID NO: 28 | 0.0% | 27.9% | 0.47 |
| Met-PRT918 | SEQ ID NO: 29 | 0.0% | 27.9% | 0.45 |
| Met-PRT699 | SEQ ID NO: 30 | 3.6% | 26.4% | -0.78 |
| Met-PRT698 | SEQ ID NO: 31 | 0.0% | 26.4% | -0.03 |
| Met-PRT966 | SEQ ID NO: 32 | 0.0% | 28.0% | 0.35 |
| Met-PRT917 | SEQ ID NO: 41 | 0.0% | 27.9% | 0.46 |
| Met-PRT1028 | SEQ ID NO: 42 | 0.0% | 28.1% | 0.05 |

The artificial fibroin of (6-i) may consist of the amino acid sequence set forth in SEQ ID NO: 25, SEQ ID NO: 26, SEQ ID NO: 27, SEQ ID NO: 28, SEQ ID NO: 29, SEQ ID NO: 30, SEQ ID NO: 31, SEQ ID NO: 32, SEQ ID NO: 41, or SEQ ID NO: 42.

The artificial fibroin of (6-ii) contains an amino acid sequence having a sequence identity of 90% or more with the amino acid sequence set forth in SEQ ID NO: 25, SEQ ID NO: 26, SEQ ID NO: 27, SEQ ID NO: 28, SEQ ID NO: 29, SEQ ID NO: 30, SEQ ID NO: 31, SEQ ID NO: 32, SEQ ID NO: 41, or SEQ ID NO: 42. The artificial fibroin of (6-ii) is also a protein having a domain sequence represented by Formula 1: [(A)ₙ motif-REP]ₘ or Formula 2: [(A)ₙ motif-REP]ₘ-(A)ₙ motif. The sequence identity is preferably 95% or more.

The content ratio of the glutamine residues in the artificial fibroin of (6-ii) is preferably 9% or less. Furthermore, the content ratio of the GPGXX motifs in the artificial fibroin of (6-ii) is preferably 10% or more.

The sixth artificial fibroin may have a tag sequence at one or both of the N-terminal and the C-terminal thereof. This makes it possible to isolate, immobilize, detect, and visualize the artificial fibroin.

More specific examples of the artificial fibroin having a tag sequence include artificial fibroin having (6-iii) an amino acid sequence set forth in SEQ ID NO: 33 (PRT888), SEQ ID NO: 34 (PRT965), SEQ ID NO: 35 (PRT889), SEQ ID NO: 36 (PRT916), SEQ ID NO: 37 (PRT918), SEQ ID NO: 38 (PRT699), SEQ ID NO: 39 (PRT698), SEQ ID NO: 40 (PRT966), SEQ ID NO: 43 (PRT917), or SEQ ID NO: 44 (PRT1028) or artificial fibroin having (6-iv) an amino acid sequence having a sequence identity of 90% or more with the amino acid sequence set forth in SEQ ID NO: 33, SEQ ID NO: 34, SEQ ID NO: 35, SEQ ID NO: 36, SEQ ID NO: 37, SEQ ID NO: 38, SEQ ID NO: 39, SEQ ID NO: 40, SEQ ID NO: 43, or SEQ ID NO: 44.

The amino acid sequences set forth in SEQ ID NO: 33, SEQ ID NO: 34, SEQ ID NO: 35, SEQ ID NO: 36, SEQ ID NO: 37, SEQ ID NO: 38, SEQ ID NO: 39, SEQ ID NO: 40, SEQ ID NO: 43, and SEQ ID NO: 44 are obtained by adding the amino acid sequence set forth in SEQ ID NO: 11 (which has a His-tag sequence and a hinge sequence) to the N-terminal of the amino acid sequences set forth in SEQ ID NO: 25, SEQ ID NO: 26, SEQ ID NO: 27, SEQ ID NO: 28, SEQ ID NO: 29, SEQ ID NO: 30, SEQ ID NO: 31, SEQ ID NO: 32, SEQ ID NO: 41, and SEQ ID NO: 42, respectively. Since only the tag sequence is added to the N-terminal, the content ratios of glutamine residues do not change, and the content ratio of glutamine residues in each of the amino acid sequences set forth in SEQ ID NO: 33, SEQ ID NO: 34, SEQ ID NO: 35, SEQ ID NO: 36, SEQ ID NO: 37, SEQ ID NO: 38, SEQ ID NO: 39, SEQ ID NO: 40, SEQ ID NO: 43, and SEQ ID NO: 44 is 9% or less (Table 4).

**[Table 4]**

| Modified fibroin | | Content ratio of glutamine residues | Content ratio of GPGXX motif | Hydrophobic degree of REP |
|---|---|---|---|---|
| PRT888 | SEQ ID NO: 33 | 6.3% | 27.9% | -0.07 |
| PRT965 | SEQ ID NO: 34 | 0.0% | 27.9% | -0.65 |
| PRT889 | SEQ ID NO: 35 | 0.0% | 27.9% | 0.35 |
| PRT916 | SEQ ID NO: 36 | 0.0% | 27.9% | 0.47 |
| PRT918 | SEQ ID NO: 37 | 0.0% | 27.9% | 0.45 |
| PRT699 | SEQ ID NO: 38 | 3.6% | 26.4% | -0.78 |
| PRT698 | SEQ ID NO: 39 | 0.0% | 26.4% | -0.03 |
| PRT966 | SEQ ID NO: 40 | 0.0% | 28.0% | 0.35 |
| PRT917 | SEQ ID NO: 43 | 0.0% | 27.9% | 0.46 |
| PRT1028 | SEQ ID NO: 44 | 0.0% | 28.1% | 0.05 |

The artificial fibroin of (6-iii) may consist of the amino acid sequence set forth in SEQ ID NO: 33, SEQ ID NO: 34, SEQ ID NO: 35, SEQ ID NO: 36, SEQ ID NO: 37, SEQ ID NO: 38, SEQ ID NO: 39, SEQ ID NO: 40, SEQ ID NO: 43, or SEQ ID NO: 44.

The artificial fibroin of (6-iv) contains an amino acid sequence having a sequence identity of 90% or more with the amino acid sequence set forth in SEQ ID NO: 33, SEQ ID NO: 34, SEQ ID NO: 35, SEQ ID NO: 36, SEQ ID NO: 37, SEQ ID NO: 38, SEQ ID NO: 39, SEQ ID NO: 40, SEQ ID NO: 43, or SEQ ID NO: 44. The artificial fibroin of (6-iv) is also a protein having a domain sequence represented by Formula 1: [(A)ₙ motif-REP]ₘ or Formula 2: [(A)ₙ motif-REP]ₘ-(A)ₙ motif. The sequence identity is preferably 95% or more.

The content ratio of glutamine residues in the artificial fibroin of (6-iv) is preferably 9% or less. Furthermore, the content ratio of the GPGXX motifs in the artificial fibroin of (6-iv) is preferably 10% or more.

The sixth artificial fibroin may contain a secretory signal for releasing a protein produced in a recombinant protein production system to the outside of a host. A sequence of the secretory signal is designed appropriately depending on the type of the host.

The artificial fibroin may be artificial fibroin having at least two characteristics among the characteristics of the first artificial fibroin, the second artificial fibroin, the third artificial fibroin, the fourth artificial fibroin, the fifth artificial fibroin, and the sixth artificial fibroin.

The artificial fibroin may be a hydrophilic artificial fibroin from a viewpoint of ensuring excellent flame retardancy. The hydrophilic artificial fibroin is preferably, for example, artificial fibroin of which a value (average HI) calculated by determining a sum of hydropathy indices (HIs) of all amino acid residues constituting the artificial fibroin and then dividing the sum by the total number of amino acid residues is 0 or less. HI is as presented in Table 2.

Examples of the hydrophilic artificial fibroin include artificial fibroin including the amino acid sequence set forth in SEQ ID NO: 4, the amino acid sequence set forth in SEQ ID NO: 6, SEQ ID NO: 7, SEQ ID NO: 8, or SEQ ID NO: 9, the amino acid sequence set forth in SEQ ID NO: 13, SEQ ID NO: 11, SEQ ID NO: 14, or SEQ ID NO: 15, the amino acid sequence set forth in SEQ ID NO: 18, SEQ ID NO: 7, SEQ ID NO: 8, or SEQ ID NO: 9, the amino acid sequence set forth in SEQ ID NO: 17, SEQ ID NO: 11, SEQ ID NO: 14, or SEQ ID NO: 15, or the amino acid sequence set forth in SEQ ID NO: 19, SEQ ID NO: 20, or SEQ ID NO: 21.

An LOI value of the artificial fibroin according to the present embodiment may be 18 or more, 20 or more, 22 or more, 24 or more, 26 or more, 28 or more, 29 or more, or 30 or more. In the present specification, the LOI value is a value measured in accordance with the "test method of a particulate or low-melting point synthetic resin" described in "Notice No. 50 of the Office of Hazardous Materials Regulation (May, 31, 1995).

The artificial fibroin may be hydrophobic artificial fibroin from a viewpoint of obtaining high hygroscopic exothermicity. The hydrophobic artificial fibroin is preferably, for example, artificial fibroin having an average HI of more than 0.

Examples of the hydrophobic artificial fibroin include artificial fibroin including the amino acid sequence set forth in SEQ ID NO: 27, SEQ ID NO: 28, SEQ ID NO: 29, SEQ ID NO: 30, SEQ ID NO: 31, SEQ ID NO: 32, SEQ ID NO: 33, or SEQ ID NO: 43, or the amino acid sequence set forth in SEQ ID NO: 35, SEQ ID NO: 37, SEQ ID NO: 38, SEQ ID NO: 39, SEQ ID NO: 40, SEQ ID NO: 41, or SEQ ID NO: 44.

A maximum hygroscopic exothermic degree of the artificial fibroin according to the present embodiment determined according to the following formula A may be more than 0.025°C/g. maximum hygroscopic exothermic degree = {(maximum value of a sample temperature when a sample is placed in a low humidity environment until the sample temperature reaches the equilibrum, and then transferred to a high humidity environment) - (a sample temperature when a sample is place in a low humidity environment until the sample tempreature reaches the equilibrum, and then transferred to a high humidity environment)} (°C) / sample weight (g)

Note that, in Formula A, the low humidity environment means an environment with a temperature of 20°C and a relative humidity of 40%, and the high humidity environment means an environment with a temperature of 20°C and a relative humidity of 90%.

A maximum hygroscopic exothermic degree of the artificial fibroin according to the present embodiment may be 0.026°C/g or more, 0.027°C/g or more, 0.028°C/g or more, 0.029°C/g or more, 0.030°C/g or more, 0.035°C/g or more, or 0.040°C/g or more. An upper limit of the maximum hygroscopic exothermic degree is not particularly limited, but is usually 0.060°C/g or less.

### <Method for producing artificial fibroin>

Artificial fibroin (hereinafter, also simply referred to as "protein") according to any one the above embodiments can be produced by, for example, expressing a nucleic acid by a nucleic acid sequence encoding the protein and a host transformed with an expression vector having one or a plurality of regulatory sequences operably linked to the nucleic acid sequence.

A method for producing a nucleic acid encoding the artificial fibroin is not particularly limited. For example, the nucleic acid can be produced by a method in which a gene encoding natural fibroin is amplified and cloned by a polymerase chain reaction (PCR) or the like, and the amplified and cloned gene is modified by a genetic engineering method, or a method for chemically synthesizing a nucleic acid. A method for chemically synthesizing a nucleic acid is not particularly limited and, for example, genes can be chemically synthesized by a method for linking, by PCR or the like, oligonucleotides that are automatically synthesized by AKTA oligopilot plus 10/100 (GE Healthcare Japan Ltd.) or the like, based on amino acid sequence information of proteins obtained from the NCBI web database and the like. In this case, in order to facilitate purification and/or confirmation of the artificial fibroin, a nucleic acid encoding artificial fibroin consisting of an amino acid sequence obtained by adding an amino acid sequence consisting of a start codon and a His10 tag to the N-terminal of the above amino acid sequence may be synthesized.

The regulatory sequence is a sequence that controls the expression of artificial fibroin in a host (for example, a promoter, an enhancer, a ribosome binding sequence, a transcription termination sequence, or the like), and can be appropriately selected depending on the type of the host. As a promoter, an inducible promoter which functions in host cells and is capable of inducing expression of artificial fibroin may be used. The inducible promoter is a promoter that can control transcription by presence of an inducer (expression inducer), absence of a repressor molecule, or physical factors such as increase or decrease in the temperature, osmotic pressure, pH value, or the like.

The type of the expression vector is appropriately selected depending on the type of the host. Examples of the expression vector include plasmid vector, viral vector, cosmid vector, fosmid vector, and artificial chromosome vector. As the expression vector, an expression vector which can automatically replicate in a host cell or can be incorporated into a chromosome of a host and which contains a promoter at a position capable of transcribing the nucleic acid encoding the artificial fibroin is suitably used.

Both prokaryotes and eukaryotes such as yeasts, filamentous fungi, insect cells, animal cells, and plant cells are suitably used as a host.

Examples of the host of the prokaryote include bacteria belonging to the genus *Escherichia,* the genus Brevibacillus, the genus Serratia, the genus Bacillus, the genus Microbacterium, the genus Brevibacterium, the genus Corynebacterium, and the genus Pseudomonas. Examples of the microorganism belonging to the genus Escherichia include Escherichia coli. Examples of the microorganism belonging to the genus Brevibacillus include Brevibacillus agri. Examples of the microorganism belonging to the genus Serratia include Serratia liquefaciens. Examples of the microorganism belonging to the genus Bacillus include Bacillus subtilis. Examples of the microorganism belonging to the genus Microbacterium include Microbacterium ammoniaphilum. Examples of the microorganism belonging to the genus Brevibacterium include Brevibacterium divaricatum. Examples of the microorganism belonging to the genus Corynebacterium include Corynebacterium ammoniagenes. Examples of the microorganism belonging to the genus Pseudomonas include Pseudomonas putida.

In a case where a prokaryote is used as a host, examples of a vector into which a nucleic acid encoding the artificial fibroin is introduced include pBTrp2 (manufactured by Boehringer Mannheim GmbH), pGEX (manufactured by Pharmacia Corporation), and pUC18, pBluescriptII, pSupex, pET22b, pCold, pUB110, and pNCO2 (JP 2002-238569 A).

Examples of the eukaryotic host include yeast and filamentous fungi (mold or the like). Examples of the yeast include those belonging to the genera Saccharomyces, Pichia, and Schizosaccharomyces. Examples of the filamentous fungi include those belonging to the genera Aspergillus, Penicillium, and Trichoderma.

In a case where a eukaryote is used as a host, examples of a vector into which a nucleic acid encoding the artificial fibroin is introduced include YEp13 (ATCC37115) and YEp24 (ATCC37051). A method for introducing an expression vector into the host cell may employ any method as long as the method introduces DNA into the host cell. Examples thereof include a method using calcium ions [Proc. Natl. Acad. Sci. USA, 69, 2110 (1972)], an electroporation method, a spheroplast method, a protoplast method, a lithium acetate method, and a competent method.

Examples of a method for expressing the nucleic acid in a host transformed with an expression vector include not only direct expression but also secretory production and fusion protein expression according to the method described in Molecular Cloning, 2nd edition.

The artificial fibroin can be produced by, for example, culturing a host transformed with the expression vector in a culture medium, producing and accumulating the protein in the culture medium, and then collecting the artificial fibroin from the culture medium. A method for culturing the host in the culture medium can be performed according to a method generally used for culturing a host.

In a case where the host is a prokaryote such as Escherichia coli or a eukaryote such as yeast, any of a natural medium and a synthetic medium may be used as a culture medium as long as it contains a carbon source, a nitrogen source, inorganic salts, and the like which can be assimilated by the host and it is a medium capable of efficiently culturing the host.

As the carbon source, any carbon source that can be assimilated by the transformed microorganisms may be used, and it is possible to use, for example, carbohydrate such as glucose, fructose, sucrose, or molasses, starch, or starch hydrolyzates containing the carbohydrate, organic acid such as acetic acid or propionic acid, and alcohol such as ethanol or propanol. As the nitrogen source, for example, it is possible to use an ammonium salt of inorganic or organic acid such as ammonia, ammonium chloride, ammonium sulfate, ammonium acetate, or ammonium phosphate, other nitrogen-containing compounds, peptone, meat extract, yeast extract, corn steep liquor, casein hydrolyzate, soybean cake, soybean cake hydrolyzate, and various fermentative bacteria and digested products thereof. Examples of the inorganic salt include monopotassium phosphate, dipotassium phosphate, magnesium phosphate, magnesium sulfate, sodium chloride, ferrous sulfate, manganese sulfate, copper sulfate, and calcium carbonate.

Prokaryotes such as Escherichia coli or eukaryotes such as yeast are cultured under aerobic conditions by, for example, shaking or aeration and agitation in submerged culture. The culture temperature is, for example, 15 to 40°C. The culture time is typically 16 hours to 7 days. The pH of the culture medium during the culturing is preferably maintained at 3.0 to 9.0. The pH of the culture medium is adjusted using, for example, an inorganic acid, an organic acid, an alkali solution, urea, calcium carbonate, and ammonia.

In addition, antibiotics such as ampicillin and tetracycline may be added to the culture medium during the culture, if necessary. When culturing microorganisms transformed with an expression vector using an inducible promoter as a promoter, an inducer may be added to the medium, if necessary. For example, in culturing of a microorganism transformed with an expression vector using a lac promoter, isopropyl-β-D-thiogalactopyranoside may be added to the medium, and in culturing of a microorganism transformed with an expression vector using a trp promoter, indole acrylic acid may be added to the medium.

Isolation and purification of the expressed artificial fibroin can be performed by a commonly used method. For example, in a case where the protein is expressed in a state of being dissolved in the cells, the host cells are collected by centrifugation after the termination of the culture and suspended in an aqueous buffer. Then, the host cells are disrupted by an ultrasonic disintegrator, a French press, a Manton-Gaulin homogenizer, a Dyno-mill, or the like, and a cell-free extract is obtained. A purified preparation is obtained from supernatant obtained by centrifuging the cell-free extract according to one of the following methods commonly used for protein isolation and purification or according to a combination of the following methods, that is, solvent extraction, salting-out using ammonium sulfate or the like, desalting, precipitation using an organic solvent, anion exchange chromatography using a resin such as diethylaminoethyl (DEAE)-sepharose or DIAION HPA-75 (available from Mitsubishi Kasei Kogyo Kabushiki Kaisha), cation exchange chromatography using a resin such as S-sepharose FF (available from Pharmacia Corporation), hydrophobic chromatography using a resin such as butyl sepharose and phenyl sepharose, gel filtration using a molecular sieve, affinity chromatography, chromatofocusing, and electrophoresis such as isoelectric focusing phoresis.

In a case where the artificial fibroin is expressed by the formation of an insoluble matter in the cell, similarly, the host cells are recovered, disrupted and centrifuged to recover the insoluble matter of the artificial fibroin as a precipitated fraction. The recovered insoluble matter of the artificial fibroin can be solubilized with a protein denaturing agent. After this operation, a purified preparation of artificial fibroin can be obtained by the same isolation and purification method as described above. In a case where the protein is secreted extracellularly, the protein can be collected from the culture supernatant. That is, a culture supernatant can be obtained by treating the culture by a method such as centrifugation, and a purified preparation can be obtained from the culture supernatant using the same isolation and purification method as described above.

### <Method for producing artificial fibroin fiber>

In a case where the artificial fibroin fiber is an artificial fibroin filament, the artificial fibroin filament can be produced by a known spinning method. That is, for example, when an artificial fibroin filament containing artificial fibroin as a main component is produced, the artificial fibroin produced according to the method described above is first added to a solvent such as dimethyl sulfoxide (DMSO), N,N-dimethylformamide (DMF), formic acid, or hexafluoroisopropanol (HFIP) together with an inorganic salt serving as a dissolution promoter as necessary, and is dissolved therein to prepare a dope solution. Subsequently, using this dope solution, spinning is performed by a known spinning method such as wet spinning, dry spinning, dry-wet spinning, or melt spinning to obtain an artificial fibroin filament. Preferred examples of the spinning method include wet spinning and dry-wet spinning.

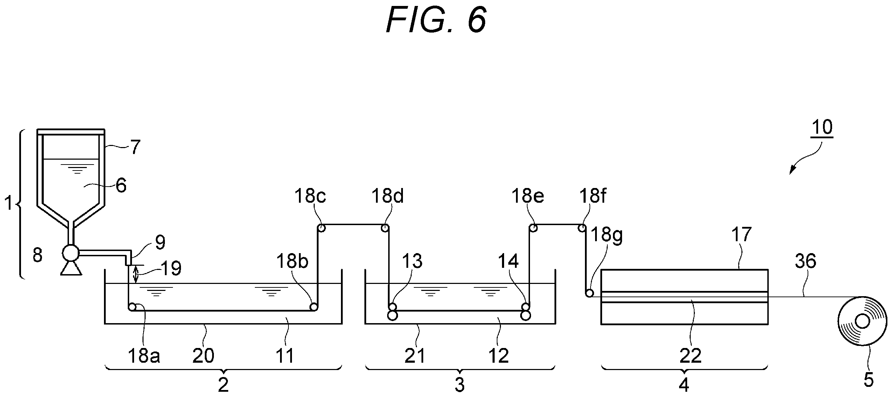

Fig. 6 is an explanatory diagram schematically illustrating an example of a spinning apparatus for producing an artificial fibroin filament. A spinning apparatus 10 illustrated in Fig. 6 is an example of a spinning apparatus for dry-wet spinning and includes an extruder 1, an undrawn yarn-producing device 2, a wet heat drawing device 3, and a drying device 4.

A spinning method using the spinning apparatus 10 will be described. First, a dope solution 6 stored in a reservoir 7 is extruded from a spinneret 9 by a gear pump 8. On a laboratory scale, a cylinder may be filled with the dope solution, and the dope solution may be extruded from a nozzle using a syringe pump. Next, the extruded dope solution 6 is fed into a coagulation liquid 11 in a coagulation liquid tank 20 through an air gap 19, a solvent is removed, and the artificial fibroin is coagulated, thus forming a fibrous coagulated body. Next, the fibrous coagulated body is fed into hot water 12 in a drawing bath 21 and drawn. A draw ratio is determined by a speed ratio between a feed nip roller 13 and a take-up nip roller 14. Thereafter, the drawn fibrous coagulated body is fed into the drying device 4 and dried in a thread guide 22, and an artificial fibroin filament 36 is obtained as a yarn package 5. Reference numerals 18a to 18g represent yarn guides.

The coagulation liquid 11 may be any solvent that can be desolvated, and examples thereof include a lower alcohol having 1 to 5 carbon atoms such as methanol, ethanol, or 2-propanol, and acetone. The coagulation liquid 11 may suitably contain water. The coagulation liquid 11 preferably has a temperature of 0°C to 30°C. In a case where a syringe pump having a nozzle with a diameter of 0.1 to 0.6 mm is used as the spinneret 9, an extrusion speed is preferably 0.2 to 6.0 mL/h, and more preferably 1.4 to 4.0 mL/h per hole. A distance in which the coagulated artificial fibroin passes through the coagulation liquid 11 (substantially, a distance from the yarn guide 18a to the yarn guide 18b) may be any length that enables efficient desolvation, and is, for example, 200 to 500 mm. A take-up speed of undrawn yarn may be, for example, 1 to 20 m/min and is preferably 1 to 3 m/min. A residence time in the coagulation liquid 11 may be, for example, 0.01 to 3 minutes and is preferably 0.05 to 0.15 minutes. Furthermore, the drawing (pre-drawing) may be performed in the coagulation liquid 11. The coagulation liquid tank 20 may be provided with multiple stages, and the drawing may be performed in each stage or in a specific stage, if necessary.

As the drawing performed when obtaining the artificial fibroin filament, for example, dry heat drawing is also adopted in addition to the pre-drawing performed in the coagulation liquid tank 20 and the wet heat drawing performed in the drawing bath 21 described above.

The wet heat drawing is performed in hot water, in a solution obtained by adding an organic solvent or the like to hot water, or with heated steam. The temperature may be, for example, 50 to 90°C and preferably 75 to 85°C. In the wet heat drawing, the undrawn yarn (or pre-drawn yarn) is drawn to, for example, 1 to 10 times and preferably 2 to 8 times its original size.

The dry heat drawing is performed using, for example, an electric tubular furnace and a dry heat plate. The temperature may be, for example, 140°C to 270°C and is preferably 160°C to 230°C. In the dry heat drawing, the undrawn yarn (or pre-drawn yarn) is drawn to, for example, 0.5 to 8 times and preferably 1 to 4 times its original size.

The wet heat drawing and the dry heat drawing may be performed independently, or performed through multiple stages, or in combination. In other words, the wet heat drawing and the dry heat drawing may be appropriately combined: for example, the wet heat drawing is performed in the first stage and the dry heat drawing in the second stage, or the wet heat drawing is performed in the first and second stages and the dry wet drawing in the third stage.

The minimum of the final draw ratio is preferably any one of over 1 time, 2 times or more, 3 times or more, 4 times or more, 5 times or more, 6 times or more, 7 times or more, 8 times or more, and 9 times or more the undrawn yarn (or pre-drawn yarn), and the maximum is preferably 40 times or less, 30 times or less, 20 times or less, 15 times or less, 14 times or less, 13 times or less, 12 times or less, 11 times or less, or 10 times or less the undrawn yarn (or pre-drawn yarn).

The length of the artificial fibroin filament obtained by the above method can be appropriately adjusted according to a spinning condition. The length of the artificial fibroin filament is not particularly limited, but can be, for example, more than 1500 m, 10,000 m or more, 15000 m or more, or 20,000 m or more.

### (Cutting step)

In a case where the artificial fibroin fiber is an artificial fibroin staple, the artificial fibroin staple can be produced by cutting an artificial fibroin filament produced by the above method. Note that the artificial fibroin filament may be cut after a crimping step or a drying step described later. The length of the artificial fibroin staple is not particularly limited, but may be 20 mm or more, 20 to 140 mm, 70 to 140 mm, or 20 to 70 mm.

### (Crimping step)

In a case where the artificial fibroin fiber is an artificial fibroin crimped fiber (an artificial fibroin crimped staple or an artificial fibroin crimped staple), the artificial fibroin crimped fiber can be produced, for example, by crimping an uncrimped artificial fibroin fiber. The artificial fibroin fiber may be crimped by conventional mechanical crimping, or may be crimped by being brought into contact with an aqueous medium. These processing methods can also be used in combination.

Examples of the mechanical crimping method include a false twist method, a pushing method, a rubbing method, an air injection method (high-pressure air jet method), and a shaping method.

The artificial fibroin fiber in the present invention shrinks and is crimped by being brought into contact with water. Therefore, according to the processing method in which the artificial fibroin fiber is brought into contact with an aqueous medium, the artificial fibroin fiber can be crimped without an external force. The aqueous medium is a liquid or gas (steam) medium containing water (including water vapor). The aqueous medium may be water or a mixed liquid of water and a hydrophilic solvent. Furthermore, as the hydrophilic solvent, for example, a volatile solvent such as ethanol or methanol, or a vapor thereof can be used. The aqueous medium is preferably a mixed solution of water and ethanol. By using an aqueous medium containing a volatile solvent or a vapor thereof, the artificial fibroin fiber is quickly dried, resulting in a softer finish. A ratio between water and the volatile solvent or a vapor thereof is not particularly limited, and for example, a mass ratio of water:volatile solvent or vapor thereof may be 10 : 90 to 90 : 10.

In a case where the aqueous medium is a liquid, for example, a known oil such as an oil for passing through a process (for example, antistatics) or an oil for finishing may be dispersed in the aqueous medium. That is, instead of the aqueous medium, an oil dispersion containing the aqueous medium and an oil dispersed in the aqueous medium can also be used. By using such an oil dispersion, the artificial fibroin fiber can be crimped, and the oil can be attached to the artificial fibroin fiber. By attaching the oil to the artificial fibroin fiber, various properties can be imparted to the artificial fibroin fiber. The amount of the oil is not particularly limited, and may be, for example, 1 to 10% by mass or 2 to 5% by mass with respect to the total amount of the aqueous medium and the oil.

A temperature of the aqueous medium may be 10°C or higher, 25°C or higher, 40°C or higher, 60°C or higher, or 100°C or higher, and may be 230°C or lower, 120°C or lower, or 100°C or lower. More specifically, in a case where the aqueous medium is a gas (steam), the temperature of the aqueous medium is preferably 100°C to 230°C and more preferably 100°C to 120°C. In a case where the steam of the aqueous medium is at a temperature of 230°C or lower, thermal denaturation of the artificial fibroin fiber can be prevented. In a case where the aqueous medium is a liquid, the temperature of the aqueous medium is preferably 10°C or higher, 25°C or higher, or 40°C or higher from a viewpoint of efficient crimping, and is preferably 60°C or lower from a viewpoint of maintaining a high fiber strength of the artificial fibroin fiber. Time during which the artificial fibroin fibers is in contact with the aqueous medium is not particularly limited, but may be 30 seconds or more, one minute or more, or two minutes or more, and is preferably 10 minutes or less from a viewpoint of productivity. The contact of the aqueous medium with the artificial fibroin fiber may be performed under normal pressure or under reduced pressure (for example, in vacuum).

Examples of a method for bringing the artificial fibroin fiber into contact with the aqueous medium include a method for immersing the artificial fibroin fiber in water, a method for spraying steam of the aqueous medium to the artificial fibroin fiber, and a method for exposing the artificial fibroin fiber to an environment filled with steam of the aqueous medium. In a case where the aqueous medium is a steam, the contact of the aqueous medium with the artificial fibroin fiber can be performed by using a general steam setting apparatus. Specific examples of the steam setting apparatus include an apparatus such as product name: FMSA-type steam setter (manufactured by FUKUSHIN KOUGYO. Co., Ltd) and product name: EPS-400 (manufactured by Tsujii Senki Kogyo Co. Ltd.). Specific examples of a method for crimping the artificial fibroin fiber using the steam of the aqueous medium include a method in which the artificial fibroin fiber is stored in a predetermined storage chamber, the steam of the aqueous medium is introduced into the storage chamber, and the steam is brought into contact with the artificial fibroin fiber while a temperature in the storage chamber is adjusted to the predetermined temperature (for example, 100°C to 230°C).

Note that the step of crimping the artificial fibroin fiber by the contact with the aqueous medium is preferably performed in a state where no tensile force is applied to the artificial fibroin fiber (no tension is applied in an axial direction of the fiber) or in a state where only a predetermined amount of tensile force is applied to the artificial fibroin fiber (only a predetermined amount of tension is applied in the axial direction of the fiber). At this time, a degree of crimping can be controlled by adjusting the tensile force applied to the artificial fibroin fiber. Examples of a method for adjusting the tensile force applied to the artificial fibroin fiber include a method for adjusting a load applied to the artificial fibroin fiber by hanging weights having various weights on the artificial fibroin fiber, a method for variously changing a degree of looseness of the artificial fibroin fiber while fixing both ends thereof in a loosened state, and a method for appropriately changing a winding force (clamping force to a paper tube or a bobbin) of the artificial fibroin fiber while the artificial fibroin fiber is wound on the paper tube or the bobbin.

### (Drying step)

After the artificial fibroin fiber is brought into contact with the aqueous medium, the artificial fibroin fiber may be dried. A drying method is not particularly limited, and natural drying may be performed, or the artificial fibroin fiber may be forcibly dried using drying equipment. The crimping by the aqueous medium and the subsequent drying can be performed continuously. Specifically, for example, the artificial fibroin fiber can be dried by being immersed in the aqueous medium while being fed from a bobbin, and then being blown with hot air or fed onto a hot roller. A drying temperature is not particularly limited, and may be, for example, 20°C to 150°C. The drying temperature is preferably 40°C to 120°C and more preferably 60°C to 100°C.

In a case where the deodorant according to the present embodiment is prepared in the form of a film, a gel, a porous body, a particle, or the like, the waterabsorbing and quick-drying property-imparting agent can be produced, for example, in accordance with the methods described in JP 2009-505668 A, JP 2009-505668 A, JP 5678283 B2, JP 4638735 B2, and the like.

### [Second Embodiment]

A second embodiment of the present invention is an agent for imparting deodorant properties, containing artificial fibroin as an active component. For the artificial fibroin, the definition of the artificial fibroin in the first embodiment can be referred to.

The agent for imparting deodorant properties can impart a deodorant effect to a target article or a raw material (material) thereof by being added to the article or the raw material (material) thereof. The description of the first embodiment can be referred to for the deodorant effect.

The shape of the agent for imparting deodorant properties according to the present embodiment is not limited as long as the agent can exhibit a deodorant effect by artificial fibroin. The agent for imparting deodorant properties is the above-described artificial fibroin itself or a composition containing the artificial fibroin. The form of the agent for imparting deodorant properties may be, for example, a liquid (for example, a solution or a suspension), a fiber, a powder, a plate, a film, a sheet, a gel, a porous body, a particle, or the like, and the shape thereof may be, for example, a cube, a rectangular parallelepiped, a triangular pyramid, a triangular prism, a cylinder, or a sphere. The agent for imparting deodorant properties imparts deodorant properties to a target article, and therefore desirably has a relatively small size as compared with the size of the target article and is light. When the agent for imparting deodorant properties is in a form of a fiber, a powder, a film, or a sheet, the agent can be used for a wider range of articles. Note that when the material for imparting deodorant properties according to the present embodiment is in a form other than a liquid, the size (thickness, area, particle size, mass, and the like) of the material is appropriately adjusted or selected according to the type of the form, the size of a target deodorant effect, and the like.

In a case where the agent for imparting deodorant properties according to the present embodiment is prepared in a form of a film, a gel, a porous body, a particle, or the like, the agent can be produced by, for example, the same method as described above similarly to the deodorant having such a form.

In a case where the agent for imparting deodorant properties is a powder, the agent may be kneaded into a raw material or a material of a target article, or may be applied to the target article (final product) at a final stage. In a case where the agent for imparting deodorant properties is a liquid, the agent may be kneaded into a raw material or a material of a target article, the raw material or the material may be impregnated with the agent, or the agent may be applied to the target article (final product) at a final stage.

In a case where the agent for imparting deodorant properties is a liquid or a powder, the agent may be contained in a container including a spray, and can be sprayed onto a target article using a volatile gas. The agent for imparting deodorant properties may be sprayed onto a raw material or a material of a target article, or may be sprayed onto the target article (final product) at a final stage.

In an embodiment, the use amount of the agent for imparting deodorant properties is preferably 3% by mass or more on the basis of the mass of a target article, and may be more than 3% by mass, 6% by mass or more, 6% by mass or more, 12% by mass or more, 15% by mass or more, 17% by mass or more, or 20% by mass or more. The use amount of the agent for imparting deodorant properties may be 70% by mass or less, 50% by mass or less, or 30% by mass or less on the basis of the mass of a target article. When the use amount of the agent for imparting deodorant properties is 3% by mass or more or more than 3%, the deodorant properties can be imparted to a predetermined article more reliably. In a case where the use amount of the agent for imparting deodorant properties is 70% or less, it is possible to reliably impart deodorant properties to a predetermined article while an increase in cost due to use of artificial fibroin is advantageously suppressed.

In a case where the agent for imparting deodorant properties is a powder, the agent may contain an optional component in addition to artificial fibroin. Examples of the optional component include an excipient, a lubricant, a binder, a stabilizer, a desiccant, a preservative, and a colorant.

In a case where the agent for imparting deodorant properties is a solution, the agent may contain an optional component in addition to artificial fibroin. Examples of the optional component include a solvent, a stabilizer, a thickener, a preservative, and a colorant.

### [Third Embodiment]

A third embodiment of the present invention is a method for imparting deodorant properties, including a step of adding artificial fibroin to an article. For the artificial fibroin, the definition of the artificial fibroin in the first embodiment can be referred to.

The method for imparting deodorant properties according to the present embodiment is a method for imparting deodorant properties to a target article by adding artificial fibroin to the target article. In a step of adding artificial fibroin to a target article, artificial fibroin itself may be added, and it is preferable to add the agent for imparting deodorant properties according to the second embodiment to the target article.

The target article is not particularly limited, and may be a composite yarn (mixed yarn), a braid, a fabric (for example, a woven fabric, a nonwoven fabric, or a knitted fabric), a covering (for example, a shirt, an undergarment, a muffler, a sock, an apron, a tie, a jacket, a coat, a glove, or a hat), bedding (for example, a sheet, a comforter, a sheet cover, a comforter cover, or a towel), or a back portion or a seat surface of a chair or a vehicle seat. Since the deodorant properties imparted by the method according to the present embodiment can mainly reduce an odor such as a sweat odor, an aging odor, or an excretion odor, the target article is preferably an article that directly touches a body.

A deodorant effect of the deodorant properties imparted by the method according to the present embodiment can be maintained even after washing is repeated 100 times. Examples

Hereinafter, the present invention will be described more specifically based on Examples. Note that the present invention is not limited to the following Examples.

### <Production of artificial spider silk fibroin>

### (1) Preparation of plasmid expression strain

Artificial fibroin having the amino acid sequence set forth in SEQ ID NO: 15 (PRT799) was designed based on the nucleotide sequence and amino acid sequence of fibroin derived from Nephila clavipes (GenBank Accession No.: P46804.1, GI: 1174415). Artificial fibroin (PRT966) having the amino acid sequence set forth in SEQ ID NO: 40 was also designed.

Next, a nucleic acid encoding the designed artificial fibroin was synthesized. In the nucleic acid, an NdeI site was added to a 5'-terminal, and an EcoRI site was added downstream of the stop codon. The nucleic acid was cloned into a cloning vector (pUC118). Thereafter, the nucleic acid was enzymatically cleaved by treatment with NdeI and EcoRI, and then, recombined into the protein expression vector pET-22b(+), thereby obtaining an expression vector.

### (2) Expression of protein

Escherichia coli BLR (DE3) was transformed with a pET22b(+) expression vector containing a nucleic acid encoding the designed artificial fibroin. The transformed Escherichia coli was cultured in 2 mL of an LB culture medium containing ampicillin for 15 hours. The culture solution was added to a 100 mL seed culture medium containing ampicillin (Table 5) such that OD₆₀₀ reached 0.005. The culture solution was maintained at a temperature of 30°C, and put in a culture flask (for about 15 hours) until OD₆₀₀ reached 5, thereby obtaining a seed culture solution.

**[Table 5]**

| Seed culture medium | |
|---|---|
| Reagent | Concentration (g/L) |
| Glucose | 5.0 |
| KH₂PO₄ | 4.0 |
| K₂HPO₄ | 9.3 |
| Yeast Extract | 6.0 |
| Ampicillin | 0.1 |

The seed culture solution was added to a jar fermenter to which a 500 mL production medium (Table 6) was added such that OD₆₀₀ was 0.05. Culture was performed while maintaining the culture solution temperature at 37°C and controlling the pH to be constant at 6.9. Furthermore, the culture solution was maintained to have a 20% dissolved oxygen concentration of the saturated dissolved oxygen concentration.

**[Table 6]**

| Production medium | |
|---|---|
| Reagent | Concentration (g/L) |
| Glucose | 12.0 |
| KH₂PO₄ | 9.0 |
| MgSO₄·7H₂O | 2.4 |
| Yeast Extract | 15.0 |
| FeSO₄·7H₂O | 0.04 |
| MnSO₄·5H₂O | 0.04 |
| CaCl₂·2H₂O | 0.04 |
| Adekanol (ADEKA, LG-295S) | 0.1 (mL/L) |

Immediately after glucose in the production medium was completely consumed, a feed solution (455 g/1 L of glucose, 120 g/1 L of Yeast Extract) was added at a speed of 1 mL/min. Culture was performed while maintaining the culture solution temperature at 37°C and controlling the pH to be constant at 6.9. The culturing was performed for 20 hours while a dissolved oxygen concentration in the culture solution was maintained at 20% of the saturated dissolved oxygen concentration. The expression of the artificial fibroin was then induced by adding 1 M isopropyl-β-thiogalactopyranoside (IPTG) to the culture solution such that the final concentration was 1 mM. When 20 hours had elapsed after the addition of IPTG, the culture solution was centrifuged, and bacterial cells were collected. SDS-PAGE was conducted using the bacterial cells prepared from the culture solution obtained before the addition of IPTG and after the addition of IPTG. The expression of the target artificial fibroin that depended on the addition of IPTG was confirmed by emergence of a band having the size of the target artificial fibroin.

### (3) Purification of Protein

Bacterial cells collected two hours after the addition of IPTG were washed with 20 mM Tris-HCl buffer (pH 7.4). The washed bacterial cells were suspended in 20 mM Tris-HCl buffer (pH 7.4) containing about 1 mM PMSF, and the cells were disrupted with a high-pressure homogenizer (available from GEA Niro Soavi). The crushed cells were centrifuged to obtain a precipitate. The obtained precipitate was washed with a 20 mM Tris-HCl buffer (pH 7.4) until the purity was high. The washed precipitate was suspended in an 8 M guanidine buffer (8 M guanidine hydrochloride, 10 mM sodium dihydrogen phosphate, 20 mM NaCl, and 1 mM Tris-HCl, pH 7.0) such that a concentration thereof was 100 mg/mL, and the suspended precipitate was dissolved by performing stirring using a stirrer at 60°C for 30 minutes. After the precipitate was dissolved, the resulting solution was dialyzed with water using a dialysis tube (cellulose tube 36/32 manufactured by Sanko Junyaku Co., Ltd.). A white coagulation protein obtained after dialysis was collected by centrifugation, and water was removed in a freeze dryer to recover freeze-dried powder, thereby obtaining artificial spider silk fibroin ("PRT799" and "PRT966").

### <Production of artificial fibroin filament>

### (1) Preparation of dope solution

The artificial fibroin (PRT799 or PRT966) described above was added to DMSO at a concentration of 24% by mass, and then LiCl was added thereto as a dissolution promoter at a concentration of 4.0% by mass. Next, the artificial fibroin was dissolved over 3 hours using a shaker to obtain a DMSO solution. Insolubles and bubbles in the obtained DMSO solution were removed to prepare a dope solution. The dope solution had a solution viscosity of 5000 cP (centipoise) at 90°C.

### (2) Spinning

Known dry-wet spinning was performed using the dope solution obtained as described above and the spinning apparatus 10 illustrated in Fig. 6 to obtain an artificial fibroin filament. The obtained artificial fibroin filament was wound around a bobbin. Note that here, dry-wet spinning was performed under the following conditions.
Temperature of coagulation liquid (methanol): 5°C to 10°C
Draw ratio: 4.52 times
Drying temperature: 80°C

### <Evaluation of deodorant effect>

### (1) Preparation of test piece

A plurality of the artificial fibroin filaments obtained as described above were bundled and cut into a length of 38 mm with a tabletop fiber cutting machine to prepare an artificial fibroin staple. The prepared artificial fibroin staple was immersed in water at 40°C for one minute to be crimped, and then dried at 40°C for 18 hours to obtain an artificial fibroin crimped staple. A shrinkage ratio of the artificial fibroin staple by immersion in water was 50%. Using the artificial fibroin crimped staple, a commercially available cotton staple, and known spinning equipment, a blended yarn of cotton: 82.5% by mass and artificial fibroin fiber: 17.5% by mass was obtained. Thereafter, by interlacing the blended yarn and a spun yarn of cotton: 100%, six types of knitted fabrics A to F each having a mixing ratio, a yarn thickness (yarn count: Nm), and a knitting pattern presented in Table 7 were prepared. Note that the knitted fabric A used a fiber spun using artificial fibroin "PRT799" as the artificial fibroin fiber, and each of the knitted fabrics B to F used a fiber spun using artificial fibroin "PRT966" as the artificial fibroin fiber. For comparison, a knitted fabric G was obtained by interlacing only a spun yarn of cotton: 100%. The thickness of the yarn (cotton count) and the knitting pattern of the knitted fabric G are the same as those of the knitted fabric A. Each of the obtained knitted fabrics was cut out in a size of 10 × 10 cm to obtain a test piece.

**[Table 7]**

| Knitted fabric | Mixing ratio | | Yarn count | Pattern |
|---|---|---|---|---|
| | Cotton | Fibroin fiber | | |
| A | 82.5 | 17.5 | 40/1 | Interlock knitting |
| B | 82.5 | 17.5 | 40/1 | Interlock knitting |
| C | 88 | 12 | 40/1 | Interlock knitting |
| D | 91 | 9 | 40/1 | Interlock knitting |
| E | 94 | 6 | 40/1 | Interlock knitting |
| F | 97 | 3 | 30/1 | Rib knitting |
| G | 100 | 0 | 40/1 | Interlock knitting |

A deodorant effect was evaluated by a method described in SEK mark textile product authentication standard (ISO17299) of the Japan Textile Evaluation Council.

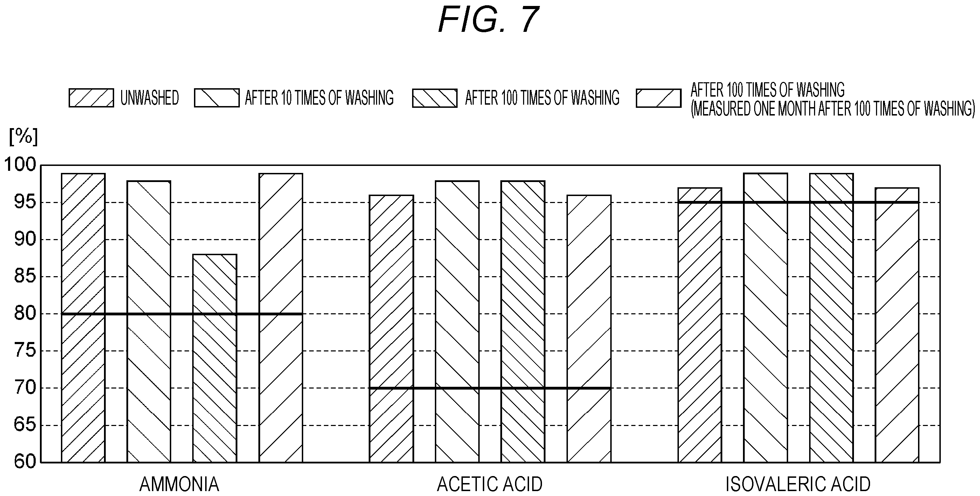

Results thereof are illustrated in Figs. 7 to 11. Fig. 7 is a graph illustrating results of evaluating deodorant properties of a fiber A. In Fig. 7, a solid line is a reference line at which it is recognized that there is a deodorant effect by an instrumental method alone. The graph in Fig. 7 illustrates, in order from the left, a deodorant effect when the knitted fabric A is unwashed, a deodorant effect after the knitted fabric A is washed 10 times, a deodorant effect after the knitted fabric A is washed 100 times, and a deodorant effect one month after the knitted fabric A is washed 100 times. The fiber A exhibited an excellent deodorant effect on ammonia, acetic acid, and isovaleric acid, and maintained the deodorant effect even after being washed 100 times.

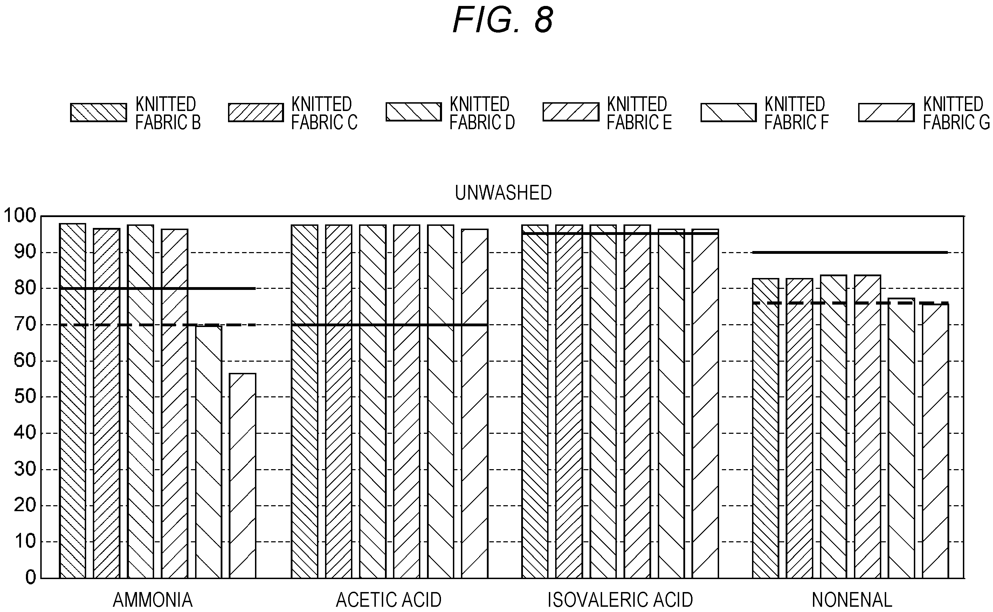

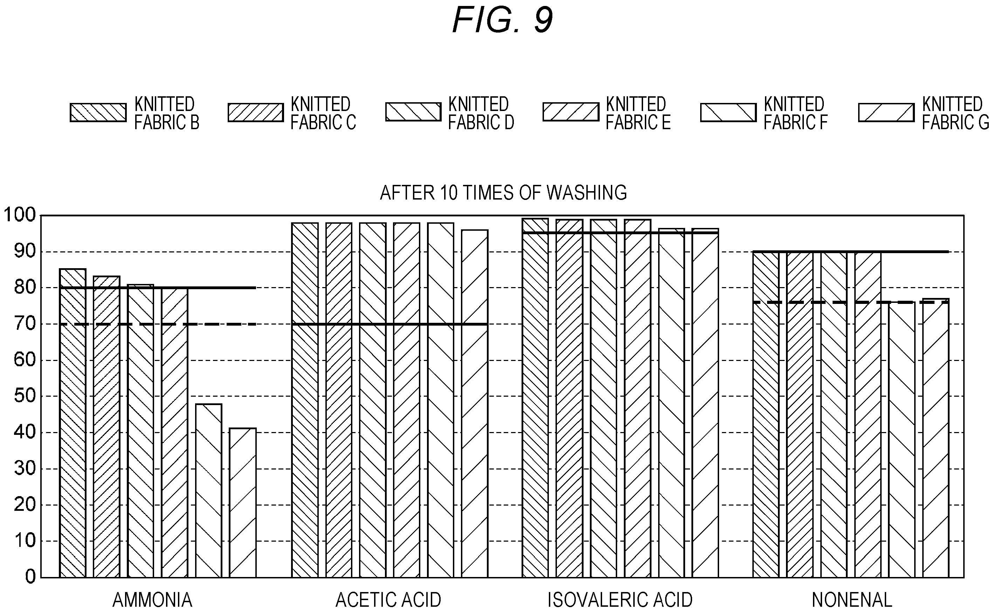

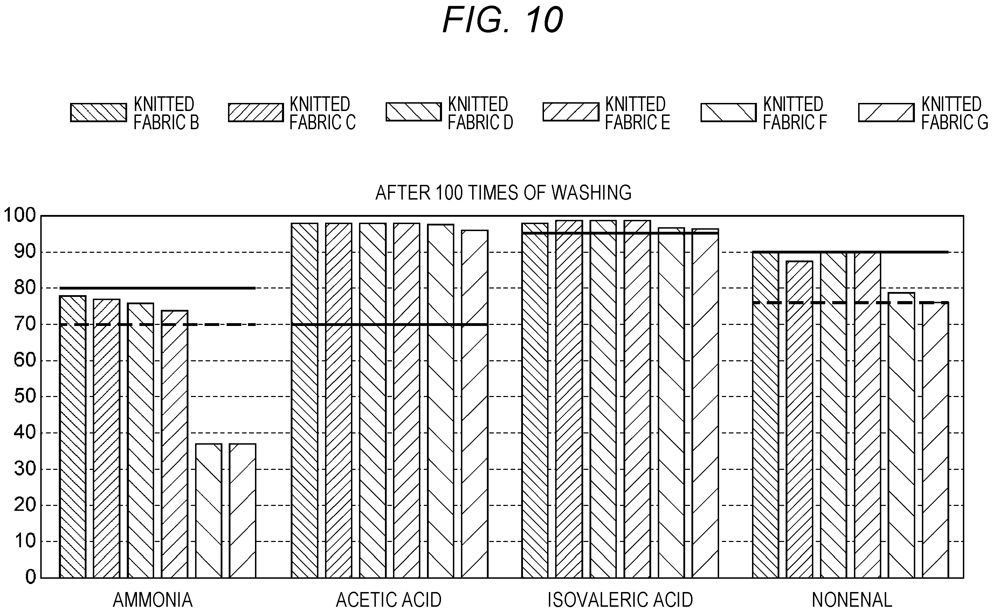

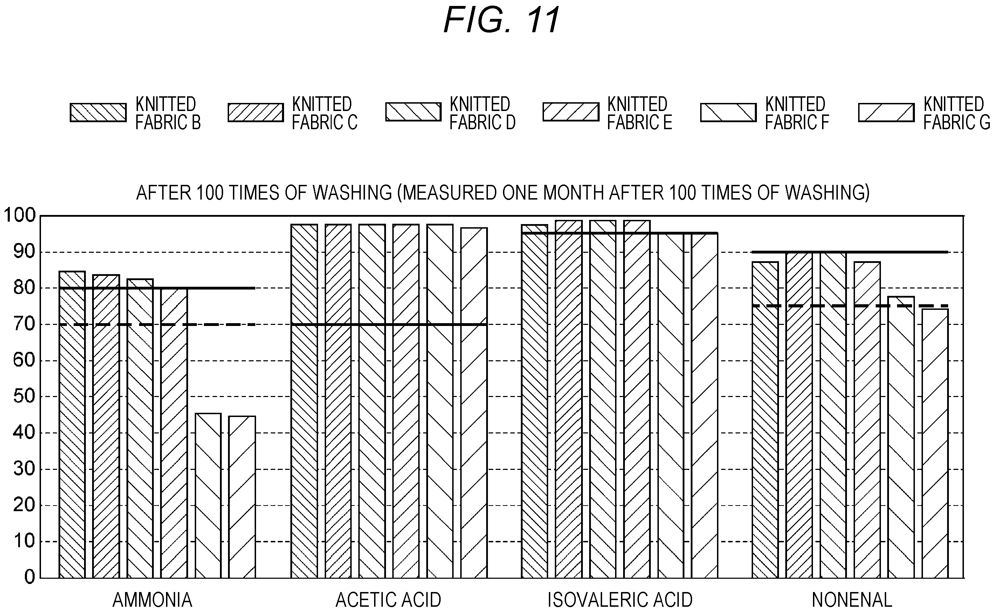

Figs. 8 to 11 are graphs illustrating deodorant effects when the knitted fabrics B to G are unwashed, deodorant effects after the knitted fabrics B to G are washed 10 times, deodorant effects after the knitted fabrics B to G are washed 100 times, and deodorant effects one month after the knitted fabrics B to G are washed 100 times, respectively. In Figs. 8 to 11, a solid line is a reference line at which it is recognized that there is a deodorant effect by an instrumental method alone, and a broken line is a reference line at which it is recognized that there is a deodorant effect in combination with a sensory test method (that is, a case where it is recognized that there is a deodorant effect by a sensory test method). In Fig. 8 (unwashed) and Fig. 9 (after 10 times of washing), the knitted fabrics B to E exhibited a sufficient deodorant effect on ammonia, acetic acid, isovaleric acid, and nonenal, and the knitted fabric F exhibited a sufficient deodorant effect on acetic acid, isovaleric acid, and nonenal. In Fig. 10 (after 100 times of washing), the knitted fabrics B and D to E exhibited a sufficient deodorant effect on acetic acid, isovaleric acid, and nonenal, and the knitted fabrics B and F exhibited a sufficient deodorant effect on acetic acid and isovaleric acid. In Fig. 11 (one month after 100 times of washing), the knitted fabrics C to D exhibited a sufficient deodorant effect on acetic acid, isovaleric acid, and nonenal, and the knitted fabrics B and E to F exhibited a sufficient deodorant effect on acetic acid and isovaleric acid. Interestingly, the knitted fabrics B to D had an insufficient deodorant effect on ammonia after 100 times of washing, but exhibited a deodorant effect on ammonia one month after 100 times of washing. The knitted fabric F exhibited an insufficient deodorant effect on ammonia and nonenal, but exhibited a deodorant effect on acetic acid and isovaleric acid. Meanwhile, Figs. 8 to 11 indicate that the knitted fabric G consisting of 100% cotton spun yarn exhibits a small deodorant effect particularly on ammonia and nonenal.

## Claims

1. A deodorant material comprising artificial fibroin as an active component.

2. The deodorant material according to claim 1, wherein the artificial fibroin contains artificial spider silk fibroin.

3. The deodorant material according to claim 1 or 2, which is in a form of a fiber.

4. An agent for imparting deodorant properties, comprising artificial fibroin as an active component.

5. The agent for imparting deodorant properties according to claim 4, wherein the artificial fibroin contains artificial spider silk fibroin.

6. The agent for imparting deodorant properties according to claim 4 or 5, which is in a form of a fiber.

7. An article comprising the agent for imparting deodorant properties according to claim 4 or 5.

8. An article comprising the agent for imparting deodorant properties according to claim 6.

9. The article according to claim 7, wherein the content of the agent for imparting deodorant properties is more than 3% by mass with respect to the total weight of the article.

10. The article according to claim 8, wherein the content of the agent for imparting deodorant properties is more than 3% by mass with respect to the total weight of the article.

11. A method for imparting deodorant properties, comprising a step of adding artificial fibroin to an article.
